(19) Europäisches Patentamt
European Patent Office
Office européen des brevets

(11) Veröffentlichungsnummer: **0 069 068**
**B1**

# (12) EUROPÄISCHE PATENTSCHRIFT

(45) Veröffentlichungstag der Patentschrift: **10.10.84**

(21) Anmeldenummer: **82810258.2**

(22) Anmeldetag: **14.06.82**

(51) Int. Cl.³: **C 07 C 69/732,** C 07 C 59/52, C 07 C 103/78, C 07 D 307/06, C 07 C 43/178, C 07 C 69/145, C 07 F 9/145, C 07 D 309/06, C 07 C 69/757, C 07 C 67/347

## (54) Hydrochinone und deren Herstellung.

(30) Priorität: **19.06.81 GB 8119015**

(43) Veröffentlichungstag der Anmeldung: **05.01.83 Patentblatt 83/1**

(45) Bekanntmachung des Hinweises auf die Patenterteilung: **10.10.84 Patentblatt 84/41**

(84) Benannte Vertragsstaaten: **BE DE FR GB IT**

(56) Entgegenhaltungen:
**EP - A - 0 025 692**
**FR - A - 2 269 333**
**US - A - 3 957 836**

(73) Patentinhaber: **CIBA-GEIGY AG, Postfach, CH-4002 Basel (CH)**

(72) Erfinder: **Howell, Frederick Harold, 27 High Bank, Atherton Lancashire M29 9HZ (GB)**

**EP 0 069 068 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Hydrochinone und ein Verfahren zu deren Herstellung. Die neuen erfindungsgemäßen Hydrochinone entsprechen der Formel I

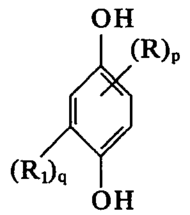

(I)

worin

p 1 oder 2 und q 0 oder 1 sind, mit der Maßgabe, daß p + q 1 oder 2 sind,
R einen Rest der Formel II

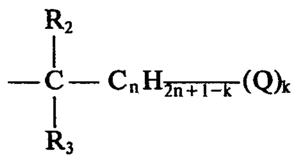

(II)

Q $-COOR_4$, $-CONR_4R_5$, $-OR_5$, $-OCOR_7$, $-NR_8R_9$, $-PO(OR_{10})([O]_xR_{11})$, $-SO_2OH$, $-SO_2Cl$, $-SO_2NR_5R_7$ oder $-CN$,
n eine ganze Zahl von 1 bis 20,
k die Zahl 1 oder 2, und
$R_1$ $C_{1-8}$-Alkyl oder einen Rest der Formel II bedeuten, wobei R und $R_1$ gleiche oder verschiedene Reste der Formel II darstellen können,
$R_2$ und $R_3$ unabhängig voneinander $C_{1-5}$-Alkyl bedeuten und, wenn $Q-COOR_4$ ist, $R_2$ oder $R_3$ durch eine Gruppe $-COOR_4$ substituiert sein können, oder $R_2$ oder $R_3$ so an den Rest $-C_nH_{2+1-k}$ gebunden sind, daß eine durch Gruppen $-(COOR_4)_k$ substituierte $C_{5-12}$-Cycloalkylengruppe gebildet wird, wobei die $R_4$ gleiche oder verschiedene Bedeutungen haben können,
$R_4$ Wasserstoff, $C_{1-20}$-Alkyl, das durch 1–5 Sauerstoffatome unterbrochen und/oder durch eine Gruppe $-OR_6$ substituiert sein kann, wobei $R_6$ $C_{1-12}$-Alkyl, $C_{3-12}$-Cycloalkyl, $C_{3-20}$-Alkenyl, $C_{7-13}$-Aralkyl oder $C_{6-10}$-Aryl bedeutet, wobei die Arylgruppe durch eine oder zwei $C_{1-4}$-Alkylgruppen substituiert sein kann, ferner $R_4$ $C_{3-20}$-Alkenyl, $C_{3-12}$-Cycloalkyl, $C_{7-13}$-Aralkyl, $C_{6-10}$-Aryl, das durch eine $C_{1-4}$-Alkylgruppe substituiert sein kann, einen 5- oder 6-gliedrigen O-haltigen Heterocyclus, der durch eine oder zwei $C_{1-4}$-Alkylgruppen substituiert sein kann, oder Methyl, das durch einen sauerstoffhaltigen 5- oder 6-gliedrigen heterocyclischen Ring substituiert ist,
$R_5$ Wasserstoff oder $C_{1-20}$-Alkyl bedeuten oder
$R_4$ und $R_5$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,
$R_7$ Wasserstoff, $C_{1-20}$-Alkyl, $C_{3-20}$-Alkenyl, $C_{3-12}$-Cycloalkyl, $C_{7-13}$-Aralkyl oder $C_{6-10}$-Aryl, das durch eine oder zwei $C_{1-4}$-Alkylgruppen substituiert sein kann, bedeutet,
$R_8$ Wasserstoff oder $C_{1-12}$-Alkyl und
$R_9$ Wasserstoff, $C_{1-12}$-Alkyl oder eine Gruppe $-COR_7$ darstellen oder
$R_8$ und $R_9$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,
x 0 oder 1,
$R_{10}$ und $R_{11}$ bei $x = 1$ unabhängig voneinander Wasserstoff oder $C_{1-20}$-Alkyl bedeuten oder $R_{10}$ und $R_{11}$ zusammen $C_{2-3}$-Alkylen bilden, das durch 1 oder mehrere $C_{1-20}$-Alkylgruppen substituiert sein kann, und bei $x = 0$
$R_{10}$ Wasserstoff oder $C_{1-20}$-Alkyl und
$R_{11}$ $C_{1-5}$-Alkyl darstellen,

mit der Maßgabe, daß $R_1$ bei $Q = -SO_2OH$ einen Rest der Formel II darstellt.

Gegenstand der Erfindung sind auch Salze von Verbindungen der Formel I mit organischen oder anorganischen Säuren und Basen.

In den obigen Definitionen können Alkyl- und Alkenylgruppen, sofern nichts anderes angegeben ist, geradkettig oder verzweigt sein.

Beispiele von geradkettigen oder verzweigten $C_{1-8}$-Alkylgruppen $R_1$ sind Methyl, Äthyl, n-Propyl, Isopropyl, n-Butyl, Isobutyl, tert.-Butyl, Neopentyl oder 1,1,3,3-Tetramethylbutyl.

Stellen $R_2$ oder $R_3$ geradkettiges oder verzweigtes $C_{1-5}$-Alkyl dar, so handelt es sich z. B. um eine Methyl-, Äthyl-, n-Propyl-, Isopropyl-, n-Butyl-, sek.-Butyl-, n-Pentyl- oder Neopentylgruppe.

Bedeutet $R_4$ geradkettiges oder verzweigtes $C_{1-20}$-Alkyl, das durch ein bis 5 Sauerstoffatome unterbrochen sein kann, so handelt es sich z. B. um eine Methyl-, Äthyl-, 2-Methoxyäthyl-, 2-Äthoxyäthyl-, 2-n-Propoxyäthyl-, 2-n-Butoxyäthyl-, n-Pentyl-, n-Decyl-, n-Heptyl-, n-Octyl-, 2-Äthylhexyl-, n-Nonyl-, n-Decyl-, n-Dodecyl-, n-Tetradecyl-, n-Hexadecyl-, n-Octadecyl-, n-Eicosyl-, $-(C_2H_4O)_2CH_3$-, $-(C_2H_4O)_3CH_3$-, $-(C_2H_4O)_4CH_3$- oder $-(C_2H_4O)_5CH_3$-Gruppe.

Beispiele von geradkettigen oder verzweigten $C_{3-20}$-Alkenylgruppen $R_4$, $R_6$ oder $R_7$ sind 2-Prop-2-enyl, n-But-2-enyl, 2-Methyl-prop-2-enyl, n-Pent-2-enyl, n-Hex-2-enyl, n-Hexa-2,4-dienyl, n-Dec-10-enyl oder n-Eicos-2-enyl. Stellen $R_4$, $R_6$ oder $R_7$ $C_{3-12}$-Cycloalkylgruppen dar, so kommen z. B. Cyclopropyl-, Cyclobutyl-, Cyclopentyl-, Cyclohexyl-, Cyclooctyl-, Cyclodecyl-, Adamantyl- oder Cyclododecylgruppen in Betracht. Als Beispiele für $C_{7-13}$-Aralkylgruppen $R_4$, $R_6$ oder $R_7$ seien genannt: die Benzyl-, Phenyläthyl-, Benzhydryl- oder Naphthylmethylgruppe.

Bedeuten $R_4$, $R_6$ oder $R_7$ $C_{6-10}$-Aryl, das durch eine geradkettige oder verzweigte $C_{1-4}$-Alkylgruppe substituiert sein kann, so handelt es sich z. B. um eine Phenyl-, Tolyl-, Xylyl-, Cumyl-, Butylphenyl- oder Naphthylgruppe.

Stellt $R_4$ einen sauerstoffhaltigen 5- oder 6-gliedrigen Heterocyclus dar, der durch eine oder zwei geradkettige oder verzweigte $C_{1-4}$-Alkylgruppen substituiert sein kann, so handelt es sich z. B. um Tetrahydrofuran-3-yl, Tetrahydropyran-4-yl oder 2,6-Dimethyl-tetrahydropyran-4-yl. Bedeutet $R_4$ Methyl, das durch einen ein Sauerstoffatom enthaltenden 5- oder 6-gliedrigen Heterocyclus substituiert ist, der seinerseits durch eine oder zwei geradkettige oder verzweigte $C_{1-4}$-Alkylgruppen substituiert sein kann, so kommen beispielsweise Furfuryl, Tetrahydrofurfuryl oder Tetrahydropyran-3-yl-methyl in Betracht.

Beispiele von geradkettigen oder verzweigten $C_{1-12}$-Alkylgruppen $R_6$, $R_8$ und $R_9$ sind Methyl, Äthyl, n-Propyl, Isopropyl, n-Butyl, sek.-Butyl, t.Amyl, 1,1,3,3-Tetramethylbutyl, n-Hexyl, n-Nonyl, n-Decyl oder n-Dodecyl. Bilden die Gruppen $R_4$ und $R_5$ oder die Gruppen $R_8$ und $R_9$ zusammen mit dem Stickstoffatom, an das sie gebunden sind, einen gegebenenfalls durch eine oder zwei $C_{1-4}$-Alkylgruppen substituierten 5- oder 6-gliedrigen heterocyclischen Ring, so handelt es sich z. B. um einen Pyrrolidin-, Piperidin-, Morpholin- oder 2,5-Dimethylmorpholinring.

Geradkettige oder verzweigte $C_{1-20}$-Alkylgruppen $R_5$, $R_7$, $R_{10}$ oder $R_{11}$ können gleich oder verschieden sein. Als Beispiele seien genannt: Methyl, Äthyl, n-Propyl, Isopropyl, n-Butyl, sek.-Butyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl oder n-Eicosyl.

Bilden $R_{10}$ und $R_{11}$ zusammen eine $C_2$- oder $C_3$-Methylenkette, die durch eine oder mehrere $C_{1-20}$-Alkylgruppen substituiert sein kann, so handelt es sich z. B. um $-CH_2CH_2$-, $-CH_2CH_2CH_2$-, $-CH_2CH(CH_3)$-, $-CH_2CH(C_2H_5)$-, $-CH_2CH(C_{20}H_{41})$-, $-CH(CH_3)CH(CH_3)$-, $-CH(CH_3)C(CH_2)_2$-, $-C(CH_3)_2C(CH_3)_2$-, $-CH_2CH_2C(CH_3)_2$- oder $-CH(CH_3)CH_2CH(CH_3)$-Gruppen.

Als Salze von Verbindungen (I), wenn Q $-COOH$ darstellt, kommen Salze mit Alkalimetallen, Erdalkalimetallen und Aminen in Betracht und, wenn Q $-NR_8R_9$ darstellt, auch Salze mit organischen oder anorganischen Basen, z. B. Chlorwasserstoff-, Schwefel-, p-Toluolsulfon- und Oxalsäure. Bevorzugt sind Verbindungen der Formel I, worin die Gruppen R und $R_1$ in 2- resp. 5-Stellung des Hydrochinonkerns stehen.

Bevorzugt sind Verbindungen der Formel III

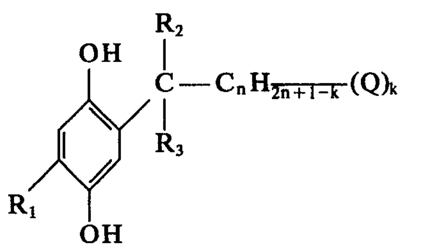

(III)

worin $R_2$, $R_3$, n, k und Q die oben angegebenen Bedeutungen haben, $R_1$ eine Gruppe der Formel II gemäß obiger Definition, oder eine Gruppe der Formel IV bedeuten

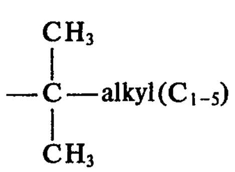

(IV)

sowie Salze davon.

Ebenfalls bevorzugt sind Verbindungen der Formel III, worin $R_1$ eine Gruppe der Formel II oder IV, Q $-COOR_4$, $-CONR_4R_5$ oder $-OR_5$ bedeuten, wobei $R_4$ und $R_5$ die oben angegebene Bedeutung haben, $R_2$ und $R_3$ unabhängig voneinander Methyl, Äthyl, n-Propyl, Isopropyl oder Neopentyl bedeuten, oder ent-

weder $R_2$ oder $R_3$ durch eine Gruppe $-COOR_4$ substituiert sein können, oder $R_2$ oder $R_3$ so an den Rest $-C_nH_{2n+1-k}$ gebunden sind, daß ein durch $-COOR_4$ substituierter $C_{5-8}$-Cycloalkylenrest gebildet wird, wobei n, k und $R_4$ die oben angegebene Bedeutung haben, sowie Salze dieser Verbindungen.

Besonders bevorzugt sind Verbindungen der Formel III, worin k die Zahl 1 ist, $R_1$ eine Gruppe der Formel II oder IV ist, Q $-COOR_4$ $-CONR_4R_5$ oder $-OR_5$ darstellt, n eine Zahl von 1 bis 10 ist, $R_2$ und $R_3$ unabhängig voneinander Methyl, Äthyl oder Neopentyl darstellen, oder entweder $R_2$ oder R3 so an den Rest $C_nH_{2n+1-k}$ gebunden sind, daß ein durch $-COOR_4$ substituierter Cyclohexylrest gebildet wird, $R_4$ Wasserstoff oder $C_{1-20}$-Alkyl bedeutet, das durch 1 bis 3 Sauerstoffatome unterbrochen und/oder durch eine Gruppe $-OR_6$ substituiert sein kann, wobei $R_6$ Cyclopentyl, Cyclohexyl, Cyclooctyl, $C_{3-10}$-Alkenyl, Phenyl, Benzyl, Phenäthyl, Benzhydryl oder Naphthylmethyl bedeutet, ferner $R_4$ $C_{3-15}$-Alkenyl, Phenyl, das durch 1 oder zwei $C_{1-4}$-Alkylgruppen substituiert sein kann, Benzyl, Phenäthyl, Cyclopentyl, Cyclohexyl oder einen sauerstoffhaltigen 5- oder 6-gliedrigen heterocyclischen Ring bedeutet, der durch eine oder zwei $C_{1-4}$-Alkylgruppen substituiert sein kann und $R_5$ die oben angegebene Bedeutung hat, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann, sowie Salze dieser Verbindungen.

Weitere bevorzugte Verbindungen entsprechen der Formel III, worin k die Zahl 1 ist, $R_1$ eine Gruppe der Formel II oder IV bedeutet, Q $-COOR_4$, $-CONR_4R_5$ oder $-OR_5$ darstellt, n eine Zahl von 1 bis 10 ist, $R_2$ und $R_3$ unabhängig voneinander Methyl, Äthyl oder Neopentyl bedeuten oder entweder $R_2$ oder $R_3$ so an den Rest $C_nH_{2n+1-k}$ gebunden sind, daß ein durch $-COOR_4$ substituierter Cyclohexylrest gebildet wird, $R_4$ Wasserstoff, $C_{1-20}$-Alkyl, das durch 1 oder 2 Sauerstoffatome unterbrochen und/oder durch Cyclohexyloxy, $C_{3-10}$-Alkenyloxy, Phenoxy oder Benzyloxy substituiert sein kann, ferner $R_4$ $C_{3-15}$-Alkenyl, Phenyl, Benzyl, Phenäthyl, Cyclopentyl, Cyclohexyl, Tetrahydrofuran-3-yl, Tetrahydropyran-4-yl oder Tetrahydrofurfuryl, einen sauerstoffhaltigen 5- oder 6-gliedrigen heterocyclischen Ring, oder Methyl, das durch einen sauerstoffhaltigen 5- oder 6-gliedrigen heterocyclischen Ring substituiert sein kann, und $R_5$ Wasserstoff oder $C_{1-15}$-Alkyl bedeutet, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann, sowie Salze davon.

Besonders bevorzugt sind Verbindungen der Formel III, worin k die Zahl 1, $R_1$ eine Gruppe der Formel II oder IV ist, Q $-COOR_4$, $-CONR_4R_5$ oder $-OR_5$ darstellt, n eine Zahl von 1 bis 3 ist, $R_2$ und $R_3$ Methyl oder Neopentyl darstellen, $R_4$ Wasserstoff, $C_{1-16}$-Alkyl, das durch ein Sauerstoffatom unterbrochen oder durch Phenoxy substituiert sein kann, Phenyl, Benzyl oder Tetrahydrofuran-3-yl bedeutet, $R_5$ Wasserstoff oder $C_{1-15}$-Alkyl ist, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann, mit der Maßgabe, daß, wenn Q $-OR_5$ darstellt, $R_5$ insbesondere $C_{1-8}$-Alkyl bedeutet, sowie Salze davon.

Als nicht limitierende Beispiele von Verbindungen der Formel I seien genannt:

Methyl-3-(2',5'-dihydroxyphenyl)-3-methyl-butyrat,
n-Hexyl-3-(2',5'-dihydroxyphenyl)-3-methyl-butyrat,
n-Hexyl-3-(4'-t-butyl-2',5'-dihydroxyphenyl)-3-methyl-butyrat,
n-Dodecyl-3-(4'-t-butyl-2',5'-dihydroxyphenyl)-3-methyl-butyrat,
2',5'-Bis-(3-methoxycarbonyl-2-methylprop-2-yl)-hydrochinon,
2',5'-Bis-(3-n-hexyloxycarbonyl-2-methylprop-2-yl)-hydrochinon,
2',5'-Bis-(3-n-dodecyloxycarbonyl-2-methylprop-2-yl)-hydrochinon,
1-(2',5'-Dihydroxyphenyl)-4-methoxycarbonyl-1-methyl-cyclohexan,
1-(2',5'-Dihydroxyphenyl)-4-n-hexyloxycarbonyl-1-methyl-cyclohexan,
2',5'-Bis-(4-methoxycarbonyl-1-methyl-cyclohex-1-yl)-hydrochinon,
2',5'-Bis-(4-n-hexyloxycarbonyl-1-methyl-cyclohex-1-yl)-hydrochinon,
2'-(5-Methoxycarbonyl-2-methyl-pent-2-yl)-hydrochinon,
2'-(5-n-Hexyloxycarbonyl-2-methyl-pent-2-yl)-hydrochinon,
2'-(5-n-Hexyloxycarbonyl-2-methyl-pent-2-yl)-5'-methyl-hydrochinon,
2'-(5-n-Hexyloxycarbonyl-2-methyl-pent-2-yl)-6'-methyl-hydrochinon,
2'-(5-Methoxycarbonyl-2-methyl-pent-2-yl)-5'-tert.-butyl-hydrochinon,
2'-(5-n-Hexyloxycarbonyl-2-methyl-pent-2-yl)-5'-tert.-butyl-hydrochinon,
2'-(5-n-Dodecyloxycarbonyl-2-methyl-pent-2-yl)-5'-tert.-butylhydrochinon,
2'-(5-Methoxycarbonyl-2-methyl-pent-2-yl)-5'-(1,1,3,3-tetramethylbutyl)-hydrochinon,
2',5'-Bis-(5-methoxycarbonyl-2-methyl-pent-2-yl)-hydrochinon,
2',5'-Bis-(5-methoxycarbonyl-2-methyl-pent-2-yl)-hydrochinon,
2',5'-Bis-(5-n-propyloxycarbonyl-2-methyl-pent-2-yl)-hydrochinon,
2'-(5-n-Hexyloxycarbonyl-2-methyl-pent-2-yl)-5'-(3-n-hexyloxycarbonyl-2-methyl-prop-2-yl)-hydrochinon,
2'-(5-n-Hexyloxycarbonyl-2-methyl-pent-2-yl)-5'-(3-n-octyloxycarbonyl-2-methyl-prop-2-yl)-hydrochinon,
2',5'-Bis-(5-iso-propyloxycarbonyl-2-methyl-pent-2-yl)-hydrochinon,

2',5'-Bis-(5 n-butyloxycarbonyl-2-methyl-pent-2-yl)-hydrochinon,
2',5'-Bis-(5-iso-butyloxycarbonyl-2-methyl-pent-2-yl)-hydrochinon,
2',5'-Bis-(5-n-pentyloxycarbonyl-2-methyl-pent-2-yl)-hydrochinon,
2',5'-Bis-(5-iso-pentyloxycarbonyl-2-methyl-pent-2-yl)-hydrochinon,
2',5'-Bis-(5-n-hexyloxycarbonyl-2-methyl-pent-2-yl)-hydrochinon,
2',5'-Bis-(5-heptyloxycarbonyl-2-methyl-pent-2-yl)-hydrochinon,
2',5'-Bis-(5-cyclohexyloxycarbonyl-2-methyl-pent-2-yl)-hydrochinon,
2',5'-Bis-(5-n-octyloxycarbonyl-2-methyl-pent-2-yl)-hydrochinon,
2',5'-Bis-5-(2-äthylhexyloxycarbonyl-2-methyl-pent-2-yl)-hydrochinon,
2',5'-Bis-(5-n-dodecyloxycarbonyl-2-methyl-pent-2-yl)-hydrochinon,
2',5'-Bis-(5-n-hexadecyloxycarbonyl-2-methyl-pent-2-yl)-hydrochinon,
2',5'-Bis-[5-(2-methoxyäthoxycarbonyl)-2-methyl-pent-2-yl]-hydrochinon,
2',5'-Bis-[5-(2-n-butyloxyäthoxycarbonyl)-2-methyl-pent-2-yl]-hydrochinon,
2',5'-Bis-[5-(2-cyclohexyloxyäthoxycarbonyl)-2-methyl-pent-2-yl]-hydrochinon,
2',5'-Bis-[5-(2-allyloxyäthoxycarbonyl)-2-methyl-pent-2-yl]-hydrochinon,
2',5'-Bis-[5-(2-benzyloxyäthoxycarbonyl)-2-methyl-pent-2-yl]-hydrochinon,
2',5'-Bis-[5-(2-phenoxyäthoxycarbonyl)-2-methyl-pent-2-yl]-hydrochinon,
2',5'-Bis-(5-phenoxycarbonyl-2-methyl-pent-2-yl)-hydrochinon,
2',5'-Bis-(5-benzyloxycarbonyl-2-methyl-pent-2-yl)-hydrochinon,
2',5'-Bis-(5-tetrahydrofurfuryloxycarbonyl-2-methyl-pent-2-yl)-hydrochinon,
2',5'-Bis-(5-furfuryloxycarbonyl-2-methyl-pent-2-yl)-hydrochinon,
2',5'-Bis-(5-tetrahydropyran-4-yloxycarbonyl-2-methyl-pent-2-yl)-hydrochinon,
2'-(5-n-Hexyloxycarbonyl-2-methyl-pent-yl)-5'-(5-methoxycarbonyl-2-methyl-pent-2-yl)-hydrochinon,
2',5'-Bis-(5-carboxy-2-methylpent-2-yl)-hydrochinon und dessen Natriumsalz,
2',5'-Bis-(5-carbamoyl-2-methylpent-2-yl)-hydrochinon,
2',5'-Bis-(5-N-n-butylcarbamoyl-2-methylpent-2-yl)-hydrochinon,
2',5'-Bis-(5-N,N-dimethylcarbamoyl-2-methylpent-2-yl)-hydrochinon,
2',5'-Bis-(5-N,N-di-n-butylcarbamoyl-2-methylpent-2-yl)-hydrochinon,
2',5'-Bis-(5-N-eicosylcarbamoyl-2-methylpent-2-yl)-hydrochinon,
2',5'-Bis-(5-N-allylcarbamoyl-2-methylpent-2-yl)-hydrochinon,
2',5'-Bis-(5-N-cyclohexylcarbamoyl-2-methylpent-2-yl)-hydrochinon,
2',5'-Bis-(5-N-benzylcarbamoyl-2-methylpent-2-yl)-hydrochinon,
2',5'-Bis-(5-N-phenylcarbamoyl-2-methylpent-2-yl)-hydrochinon,
2',5'-Bis-(5-morpholinocarbamoyl-2-methylpent-2-yl)-hydrochinon,
2'-(7-Methoxycarbonyl-2,2,4-trimethyl-hept-4-yl)-5'-tert.-butylhydrochinon,
Dimethyl-5-methyl-5-(2',5'-dihydroxyphenyl)-azelat,
2',5'-Bis-(2,6-dimethyl-8-hydroxy-oct-2-yl)-hydrochinon,
2',5'-Bis-(8-acetyloxy-2,6-dimethyl-oct-2-yl)-hydrochinon,
2',5'-Bis-(2,6-dimethyl-8-propionyloxy-oct-2-yl)-hydrochinon,
2',5'-Bis-(8-butyryloxy-2,6-dimethyl-oct-2-yl)-hydrochinon,
2',5'-Bis-(2,6-dimethyl-8-hexanoyloxy-oct-2-yl)-hydrochinon,
Diäthyl-5-(4'-tert.-butyl-2',5'-dihydroxyphenyl)-2-äthoxycarbonyl-5-methylhexan-2-phosphonat,
2',5'-Bis(5,5-diäthoxycarbonyl-2-methyl-pent-2-yl)-hydrochinon,
2',5'-Bis-(2,6-dimethyl-8-eicosanoyloxy-oct-2-yl)-hydrochinon,
2',5'-Bis-(8-crotonoyloxy-2,6-dimethyl-oct-2-yl)-hydrochinon,
2',5'-Bis-(8-benzoyloxy-2,6-dimethyl-oct-2-yl)-hydrochinon,
2',5'-Bis-(2,6-dimethyl-8-phenacetyloxy-oct-2-yl)-hydrochinon,
2',5'-Bis-(8-cyclohexylcarbonyloxy-2,6-dimethyl-oct-2-yl)-hydrochinon,
2',5'-Bis-(8-methoxy-2,6-dimethyl-oct-2-yl)-hydrochinon,
2',5'-Bis-(8-n-butoxy-2,6-dimethyl-2-yl)-hydrochinon,
2',5'-Bis-(6-amino-2-methyl-hept-2-yl)-hydrochinon und dessen Hydrochlorid,
2',5'-Bis-(6-N-methylamino-2-methyl-hept-2-yl)-hydrochinon,
2',5'-Bis-(6-N,N-dimethylamino-2-methyl-hept-2-yl)-hydrochinon,
2',5'-Bis-(6-N-äthylamino-2-methyl-hept-2-yl)-hydrochinon,
2',5'-Bis-(6-N,N-diäthylamino-2-methyl-hept-2-yl)-hydrochinon,
2',5'-Bis-(6-N-butylamino-2-methyl-hept-2-yl)-hydrochinon,
2',5'-Bis-(6-N,N-di-n-butylamino-2-methyl-hept-2-yl)-hydrochinon,
2',5'-Bis-(2-methyl-6-morpholino-hept-2-yl)-hydrochinon,
2',5'-Bis-(6-acetamido-2-methyl-hept-2-yl)-hydrochinon,
2'-(6-Acetamido-2-methyl-hept-2-yl)-hydrochinon,
2',5'-Bis-(6-hexanamido-2-methyl-hept-2-yl)-hydrochinon,
2',5'-Bis-(12-amino-2,13-dimethyl-tetradec-2-yl)-hydrochinon,
2',5'-Bis-(12-amino-3,13-dimethyl-tetradec-3-yl)-hydrochinon,

2',5'-Bis-(12-acetamido-2,13-dimethyl-tetradec-2-yl)-hydrochinon,
2',5'-Bis-(12-acetamido-3,13-dimethyl-tetradec-3-yl)-hydrochinon,
3-(2',5'-Dihydroxyphenyl)-3-methyl-butan-phosphonsäure und deren Natriumsalz,
2',5'-Bis-(2-methyl-4-phosphono-but-2-yl)-hydrochinon,
Dimethyl-3-(2',5'-dihydroxyphenyl)-3-methyl-butan-phosphonat,
Diäthyl-3-(2',5'-dihydroxyphenyl)-3-methyl-butan-phosphonat,
Di-n-butyl-3-(2',5'-dihydroxyphenyl)-3-methyl-butan-phosphonat,
Dimethyl-3-(4'-t-butyl-2',5'-dihydroxyphenyl)-3-methyl-butan-phosphonat,
Dimethyl-2-[4'-(1,1,3,3-tetramethylbutyl-2',5'-dihydroxyphenyl]-3-methyl-butan-phosphonat,
2',5'-Bis-(2-methyl-4-dimethylphosphono-but-2-yl)-hydrochinon,
2',5'-Bis-(2-methyl-4-diäthylphosphono-but-2-yl)-hydrochinon,
2',5'-Bis-(2-methyl-4-di-n-propylphosphono-but-2-yl)-hydrochinon,
2'-(5-n-Hexyloxycarbonyl-2-methyl-pent-2-yl)-5'-(2-methyl-4-diäthyl-phosphono-but-2-yl)-hydrochinon,
2',5'-Bis-(2-methyl-4-di-iso-propylphosphono-but-2-yl)-hydrochinon,
2',5'-Bis-(2-methyl-4-di-n-butyl-phosphono-but-2-yl)-hydrochinon,
2',5'-Bis-[2-methyl-4-(di-2-äthylhexylphosphono)-but-2-yl]-hydrochinon,
2',5'-Bis-(2-methyl-4-di-n-dodecylphosphono-but-2-yl)-hydrochinon,
2',5'-Bis-[2-methyl-4-(2-oxo-1,3,2-dioxaphospholan-2-yl)-but-2-yl]-hydrochinon,
2',5'-Bis-[2-methyl-4-(4-methyl-2-oxo-1,3,2-dioxaphospholan-2-yl)-but-2-yl]-hydrochinon,
2',5'-Bis-[4-(äthyl-äthylphosphino)-2-methyl-but-2-yl]-hydrochinon,
2',5'-Bis-(1-methyl-3-sulfo-prop-2-yl)-hydrochinon,
2-(2',5'-Dihydroxyphenyl)-2-methyl-propansulfonamid,
2-(4'-t-Butyl-2',5'-dihydroxyphenyl)-2-methyl-propansulfonamid,
N-Methyl-2-(2',5'-dihydroxyphenyl)-2-methylpropansulfonamid,
N-Methyl-2-[2',5'-dihydroxy-4'-(1,1,3,3-tetramethylbutyl)-phenyl]-2-methyl-propansulfonamid,
N-N-Di-n-butyl-2-(2',5'-dihydroxyphenyl)-2-methylpropansulfonamid,
N-n-Octyl-2-(2',5'-dihydroxyphenyl)-2-methylpropansulfonamid,
N-n-Octyl-2-(4'-tert.-butyl-2',5'-dihydroxyphenyl)-2-methylpropansulfonamid,
2',5'-Bis-(5-cyano-2-methyl-pent-2-yl)-hydrochinon.

Gegenstand der Erfindung ist auch ein Verfahren zur Herstellung von Verbindungen der Formel I und definitionsgemäßen Salzen davon, indem man in Gegenwart eines sauren Katalysators ein Hydrochinon der Formel V

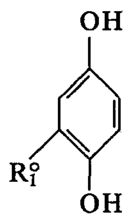

(V)

mit einem funktionellen Alkylierungsmittel (VI), das zur Einführung einer Gruppe der Formel II befähigt ist, umsetzt. Dabei stellt $R_1^o$ Wasserstoff oder $R_1$ dar.

Die Alkylierung wird zweckmäßig bei einer Temperatur zwischen 20 und 150°C, bevorzugt zwischen 80 und 130°C, durchgeführt. Der saure Katalysator kann eine Brønsted- oder Lewis-Säure oder eine aktive Erde sein. Für diesen Zweck geeignete Brønsted-Säuren können organisch oder anorganisch oder teilweise Salze davon sein. In Betracht kommen z. B. anorganische Mineralsäuren, wie Chlorwasserstoffsäure, Schwefel-, Perchlor- und Orthophosphorsäure; alkyl-, aryl- oder aralkyl-substituierte anorganische Säuren, wie Methan- und Äthansulfonsäure, Benzolsulfonsäure, p-Toluolsulfonsäure und Methanphosphonsäure; organische Säuren, wie Dichloressigsäure, Trichloressigsäure und Trifluoressigsäure. Für die Alkylierung geeignete Lewis-Säuren sind z. B. Bortrifluorid, Eisen(III)chlorid, Aluminiumchlorid und Zinn(IV)chlorid. Für die Alkylierung geeignete aktive Erden sind z. B. Fulmont® 237 und Fulcat® 22.

p-Toluolsulfonsäure wird als Alkylierungskatalysator bevorzugt. Verbindungen der Formel I, worin q 0 oder 1 und p 1 sind, können aus Verbindungen der Formel V, worin $R_1^o$ Wasserstoff, $C_{1-8}$-Alkyl oder einen Rest der Formel II darstellt, und 0,1 bis 1,0 Mol eines Alkylierungsmittels (VI) pro Mol Verbindung der Formel V erhalten werden. Andererseits können Verbindungen der Formel I, worin q 1 ist und $R_1$ $C_{4-8}$-Alkyl darstellt, durch Alkylierung von Verbindungen der Formel I, worin q 0 und p 1 sind, mit mindestens einem Mol eines $C_{4-8}$-Olefins oder -Alkohols in Gegenwart eines sauren Katalysators hergestellt werden. Verbindungen der Formel I, worin q 0 und p 2 sind und worin die zwei einzuführenden Gruppen der Formel II gleich sind, können aus Verbindungen der Formel V mit $R_1^o$ = H und mindestens 2 Mol eines Alkylierungsmittels (VI) pro Mol der Verbindung der Formel V hergestellt werden.

Beispiele von Hydrochinonen der Formel V sind: Hydrochinon, Toluylhydrochinon, 2-Äthylhydrochinon, 2-Propylhydrochinon, 2-Isopropylhydrochinon, 2-n-Butylhydrochinon, 2-Isobutylhydrochinon, 2-sek.-Butylhydrochinon, 2-tert.-Butylhydrochinon, 2-(1,1,3,3-Tetramethylbutyl)-hydrochinon.

Die funktionellen Alkylierungsmittel VI, die mit den Hydrochinonen der Formel V umgesetzt werden, enthalten ein aktives Zentrum, z. B. eine olefinische Gruppe oder eine Hydroxylgruppe, die während der Alkylierungsreaktion eliminiert, verändert oder umgelagert wird.

Beispiele von funktionellen Olefinen, die sich zur Alkylierung von Verbindungen der Formel V eignen, sind: 5-Methylhex-5-ensäure, 5-Methyl-hex-5-ensäuremethylester, 5-Methylhex-5-ensäureäthylester, 5-Methylhex-5-ensäure-n-propylester, -isopropylester, -n-butylester, -isobutylester, -sek.-butylester, -n-pentylester, -2-methylbutylester, -2,2-dimethylpropylester, -n-hexylester, -cyclohexylester, -2-äthylhexylester, -n-octylester, -n-dodecylester oder -n-hexadecylester, 5,7,7-Trimethyl-oct-4-ensäuremethylester, 1,7-Dimethoxycarbonyl-4-methyl-hept-3-en, 6-Methoxycarbonyl-1-methyl-cyclohex-1-en, Dimethylprenylphosphonat, Diäthylprenylphosphonat, Di-n-propylprenylphosphonat, Di-isopropylprenylphosphonat, Di-n-butylprenylphosphonat, Di-n-octylprenylphosphonat, Citronellol, Citronellylacetat, Citronellyl-methyläther, Citronellyl-butyläther, 2-Amino-6-methyl-hept-5-en, 2-Amino-6-methyl-hept-6-en, Diäthyl-2-äthoxycarbonyl-5-methyl-hex-4-en-2-phosphonat, 2-Äthoxycarbonyl-5-methyl-hex-4-ensäureäthylester, 2-Acetamido-6-methyl-hept-5-en, 2-Acetamido-6-methyl-hept-6-en, 2-Methyl-1-propen-1-sulfonsäure, 2-Methyl-2-propen-1-sulfonsäureamid, N-n-Butyl-2-propen-1-sulfonsäureamid, N,N-Di-n-butyl-2-propen-1-sulfonsäureamid, N-n-Octyl-2-propen-1-sulfonsäureamid.

Beispiele von funktionellen Hydroxyverbindungen für die funktionelle Alkylierung von Verbindungen der Formel V sind: 2-Amino-6-hydroxy-6-methylheptan, 2-Acetamido-6-hydroxy-6-methylheptan, 11-Amino-2,2,12-trimethyl-tridecan-1-ol, sowie aus 11-Aminoundecanolen der Formel VI a

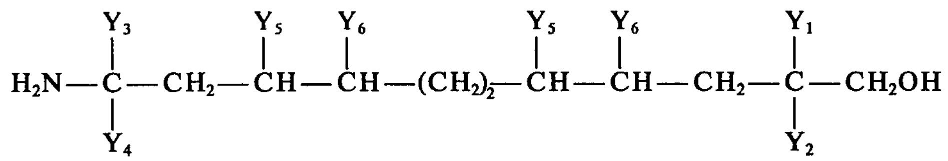

(VI a)

ausgewählte Verbindungen, worin $Y_1$ und $Y_3$ unabhängig voneinander Wasserstoff oder $C_{1-8}$-Alkyl, $Y_2$ und $Y_4$ unabhängig voneinander $C_{1-8}$-Alkyl und $Y_5$ und $Y_6$ unabhängig voneinander Wasserstoff oder $C_{1-4}$- Alkyl bedeuten. Derartige 11-Aminoundecanole und deren Herstellung sind ausführlich in der DE-OS 2 831 299 beschrieben.

Beispiele von für die Alkylierung von Verbindungen der Formel I mit $q = 0$ und $p = 1$ geeigneten Olefinen sind Isobutylen und Diisobutylen. Als Alkohole für die Alkylierung von Verbindungen der Formel I mit $q = 0$ und $p = 1$ eignen sich zum Beispiel tert.-Butanol und 1,1,3,3-Tetramethyl-butan-1-ol. Die funktionellen Derivate der Formel I können auch in andere funktionelle Derivate übergeführt werden. Stellt Q z. B. $-COOH$ dar, so kann diese Gruppe mit einem Alkohol $R^4-OH$ zum entsprechenden Ester $-COOR^4$ umgesetzt werden. Estergruppen $Q = -COOR^4$ können durch Umesterung in andere Gruppen $R^4$ übergeführt werden, oder Estergruppen $-COOR^4$ können durch Behandlung mit Verbindungen $NHR^4R^5$, worin $R^4$ und $R^5$ die oben angegebene Bedeutung haben, in Amide $-CONR^4R^5$ übergeführt werden.

Geeignete Katalysatoren für eine derartige Veresterung sind beispielsweise p-Toluolsulfonsäure und Camphersulfonsäure.

Mit der vorliegenden Erfindung wird die Einführung von verschiedenartigen funktionellen Alkylestern in das Hydrochinonmolekül ermöglicht, so daß die photographische Wirkung dieses Hydrochinons optimal ist. Beispielsweise können die Polarität und/oder der Ballast in den Hydrochinonen der Formel I so eingestellt werden, daß sie für photographische Systeme eine wirkliche Kontrolle der Löslichkeit, Verträglichkeit und Beweglichkeit/Unbeweglichkeit ermöglichen. Mit derartigen Eigenschaften stellen die Hydrochinone der Formel I wertvolle Zwischenprodukte für die Herstellung von photographisch komplizierteren Verbindungen dar. Ferner sind sie als photographische Stabilisatoren verwendbar.

Die Verbindungen der Formel I eignen sich als Lichtstabilisatoren für photographische Systeme und zur Herstellung von photographischen Chemikalien. Die Hydrochinone der Formel I wie auch Farbkuppler können in bekannter Weise in photographische Schichten eingearbeitet werden, z. B. in Silberhalogenidemulsionen, die Gelatine und/oder andere Bindemittel enthalten. Sie finden z. B. Anwendung in Silberbromid-, Silberchlorid- oder Silberjodidemulsionen oder in Emulsionen, die Gemische von Silberhalogeniden enthalten, wie Silberbromid/jodid- oder Silberchlorid/bromid-Emulsionen. Die Emulsionen können chemisch sensibilisiert werden und auch übliche organische Stabilisatoren und Antischleiermittel sowie übliche Weichmacher, wie Glyzerin, enthalten. Die Emulsionen können auch mit für Gelatine üblichen Härtern gehärtet werden. Schließlich können die Emulsionen auch übliche Beschichtungshilfsmittel enthalten. Die Emulsionen können auf übliche Trägermaterialien für photographisches Bildmaterial appliziert werden. Gegebenenfalls kann ein Gemisch verschiedener Kolloide verwendet werden, um die Silberhalogenide zu dispergieren.

Zum Entwickeln des Bildmaterials für Farbphotographie können übliche Entwicklungsbäder eingesetzt werden. Diese Bilder enthalten in der Regel eine Entwicklungssubstanz des p-Phenyldiamin-Typs, einen Entwicklungsverzögerer, wie Kaliumbromid, ein Antioxidans, wie Natriumsulfit, und eine Base, z. B. ein Alkalimetallhydroxid oder Alkalimetallcarbonat. Die Entwicklungsbäder können auch übliche Antischleiermittel und Komplexiermittel enthalten.
Entsprechende Anwendungsmöglichkeiten sind z. B. in den US-Patentschriften 2 304 939, 2 304 940, 2 322 027, 2 284 879, 2 801 170, 2 801 171, 2 749 360 und 2 825 382 beschrieben.
Die erfindungsgemäßen Hydrochinone zeichnen sich gegenüber bekannten Hydrochinonen auch durch andere Vorteile aus : so haben sie z. B. eine erhöhte Stabilität gegen aktinisches Licht und können so länger gelagert werden.
In den Beispielen sind Teile und Prozente Gewichtsteile bzw. Gewichtsprozente. Der Druck wird in Bar bzw. Millibar angegeben.

Beispiel 1

110 Teile Hydrochinon, 284 Teile 5-Methylhex-5-ensäuremethylester und 10 Teile p-Toluolsulfonsäure werden 24 Stunden auf einem Dampfbad erhitzt. Das abgekühlte Reaktionsgemisch verfestigt sich zum Teil und ergibt nach dem Verreiben mit Diäthyläther 2',5'-Bis-(5-methoxycarbonyl-2-methylpent-2-yl)-hydrochinon; Smp. 150—3° C. Nach dem Kristallisieren aus Methanol/Wasser hat das Produkt einen Smp. von 160—2° C.

Analyse für $C_{22}H_{34}O_6$:

| | | |
|---|---|---|
| gefunden | C 67,05 | H 8,96%; |
| berechnet | C 66,98 | H 8,69%. |

Beispiel 2

25,0 Teile 2',5'-Bis-(5-methoxycarbonyl-2-methylpent-2-yl)-hydrochinon, 250 Teile Eisessig und 125 Teile 46%ige wäßrige Bromwasserstoffsäure werden 15 Stunden bei Raumtemperatur gelagert. Nach dieser Zeit wird 2',5'-Bis-(5-carboxy-2-methylpent-2-yl)-hydrochinon abfiltriert, das ein halbes Mol kristallisierte Essigsäure enthält und einen Smp. von 221—4° C aufweist.

Analyse für $C_{20}H_{30}O_6(CH_3COOH)1/2$:

| | | |
|---|---|---|
| gefunden | C 63,68 | H 8,38%; |
| berechnet | C 63,62 | H 8,14%. |

5,0 Teile 2',5'-Bis-(5-carboxy-2-methylpent-2-yl)-hydrochinon, 100 Teile wasserfreies Äthanol und 1,0 Teil 98%ige Schwefelsäure werden 16 Stunden am Rückfluß gehalten. Dann wird die Lösung unter vermindertem Druck abgezogen, der Rückstand in Diäthyläther aufgenommen und mit Natriumbicarbonatlösung gewaschen. Nach dem Einengen der ätherischen Lösung und dem Verdünnen mit Petroläther (Siedebereich 40—60° C) erhält man 2',5'-Bis-(5-äthoxycarbonyl-2-methyl-2-yl)-hydrochinon vom Smp. 146—9° C.

Analyse für $C_{24}H_{38}O_6$:

| | | |
|---|---|---|
| gefunden | C 68,62 | H 9,43%; |
| berechnet | C 68,22 | H 9,06%. |

Die Beispiele 3—20 in der folgenden Tabelle I illustrieren weitere Ester, die in analoger Weise wie in Beispiel 2 beschrieben aus 2',5'-Bis-(5-carboxy-2-methylpent-2-yl)-hydrochinon hergestellt werden.

Tabelle I

| Beispiel Nr. | Diester von 2',5'-Bis-(5-carboxy-2-methylpent-2-yl)-hydrochinon | Smp. °C | Molekular-Formel | Analyse (gefunden, berechnet in %) C | H |
|---|---|---|---|---|---|
| 3 | n-Propyl | 134–6 | $C_{26}H_{42}O_6$ | 69,08<br>69,30 | 9,29<br>9,36 |
| 4 | Iso-propyl | 140–3 | $C_{26}H_{42}O_6$ | 68,57<br>69,30 | 9,70<br>9,36 |
| 5 | n-Butyl | 105–8 | $C_{28}H_{46}O_6$ | 70,54<br>70,26 | 9,97<br>9,69 |
| 6 | Iso-butyl | 135–8 | $C_{28}H_{46}O_6$ | 70,57<br>70,26 | 9,97<br>9,96 |
| 7 | n-Pentyl | 80–4 | $C_{30}H_{50}O_6$ | 71,88<br>71,11 | 11,10<br>9,95 |
| 8 | Iso-pentyl | 94–7 | $C_{30}H_{50}O_6$ | 70,65<br>71,11 | 9,97<br>9,95 |
| 9 | n-Hexyl | 85–7 | $C_{32}H_{54}O_6$ | 71,77<br>71,84 | 10,23<br>10,18 |
| 10 | Cyclohexyl | 176–80 | $C_{32}H_{50}O_6$ | 72,61<br>72,42 | 9,74<br>9,50 |
| 11 | n-Heptyl | 87–8 | $C_{34}H_{58}O_6$ | 72,75<br>72,56 | 10,48<br>10,39 |
| 12 | 2-Äthylhexyl | 60–2 | $C_{36}H_{62}O_6$ | 73,15<br>73,18 | 10,76<br>10,58 |
| 13 | n-Octyl | 77–80 | $C_{36}H_{62}O_6$ | 73,39<br>73,18 | 10,71<br>10,58 |
| 14 | n-Dodecyl | 77–9 | $C_{44}H_{78}O_6$ | 75,46<br>75,21 | 10,91<br>11,11 |
| 15 | n-Hexadecyl | 81–4 | $C_{52}H_{94}O_6$ | 76,48<br>76,65 | 11,59<br>11,54 |
| 16 | Allyl | 136–9 | $C_{26}H_{38}O_6$ | 70,14<br>69,93 | 8,61<br>8,58 |
| 17 | Tetrahydrofurfuryl | 112–4 | $C_{30}H_{46}O_8$ | 67,23<br>67,39 | 8,90<br>8,67 |
| 18 | 2-n-Butoxyäthyl | 87–9 | $C_{32}H_{54}O_8$ | 67,70<br>67,81 | 9,88<br>9,60 |
| 19 | 2-Phenoxyäthyl | 140–3 | $C_{36}H_{46}O_8$ | 70,63<br>71,26 | 7,98<br>7,64 |
| 20 | Benzyl | 139–41 | $C_{34}H_{42}O_6$ | 74,57<br>74,70 | 7,89<br>7,74 |

Beispiel 21

8,0 Teile 2',5'-Bis-(5-methoxycarbonyl-2-methyl-pent-2-yl)-hydrochinon, 100 Teile Furfurylalkohol und 1,0 Teil Natriumhydroxid werden 24 Stunden auf einem Dampfbad erhitzt. Das abgekühlte Reaktionsgemisch wird mit Diäthyläther verdünnt und mit Wasser gewaschen. Nach dem Entfernen des Diäthyläthers und des überschüssigen Furfurylalkohols wird der Rückstand mit Petroläther, der bei 40—60° C siedet und noch Diäthyläther enthält, verrieben. Man erhält 2',5'-Bis-(5-furfuryloxycarbonyl-2-methyl-pent-2-yl)-hydrochinon vom Smp. 139—42° C. Die Kristallisation aus Methanol, der 5% Wasser enthält, liefert Kristalle vom Smp. 140—3° C.

Analyse für $C_{30}H_{38}O_8$:

| | | |
|---|---|---|
| gefunden | C 67,45 | H 7,50%; |
| berechnet | C 68,42 | H 7,27%. |

Beispiel 22

5,5 Teile Hydrochinon, 21,2 Teile 5-Methyl-hex-5-ensäure-n-hexylester und 1,0 Teil p-Toluolsulfonsäure werden 4 Tage auf einem Dampfbad erhitzt. Das abgekühlte Reaktionsgemisch wird in Diäthyläther aufgenommen, mit 10% Natriumhydroxidlösung gewaschen und dann mit Wasser gewaschen, bis es neutral ist. Nach dem Abziehen restlicher Lösungsmittelanteile wird das zurückgebliebene Öl, das sich teilweise verfestigt, mit Petroläther (Siedebereich 40—60° C) verrieben. Man erhält 2',5'-Bis-(5-n-hexyloxycarbonyl-2-methyl-pent-2-yl)-hydrochinon vom Smp. 77—8° C.
Durch eine weitere Kristallisation aus Petroläther vom Siedebereich 60—80° C erhält man ein Produkt vom Smp. 83—6° C, das mit der gemäß Beispiel 9 erhaltenen Verbindung identisch ist.

Beispiel 23

2,0 Teile 2',5'-Bis-(5-methoxycarbonyl-2-methyl-pent-2-yl)-hydrochinon und 15,0 Teile n-Butylamin werden 18 Stunden am Rückfluß gehalten. Überschüssiges n-Butylamin wird unter vermindertem Druck entfernt. Nach Behandeln mit Diäthyläther ergibt der Rückstand 2',5'-Bis-(5-N-n-butylcarbamoyl-2-methyl-pent-2-yl)-hydrochinon, das nach dem Kristallisieren aus Äthanol einem Smp. von 198—200° C aufweist.

Analyse für $C_{28}H_{48}N_2O_4$:

| | | | |
|---|---|---|---|
| gefunden | C 70,39 | H 10,21 | N 5,83%; |
| berechnet | C 70,55 | H 10,15 | N 5,88%. |

Beispiel 24

2,0 Teile 2',5'-Bis-(5-methoxycarbonyl-2-methyl-pent-2-yl)-hydrochinon und 10,0 Teile Di-n-butylamin werden in ein Carius-Rohr aus Glas eingeschmolzen und 72 Stunden auf 160° C erhitzt. Nach dem Entfernen des überschüssigen Di-n-butylamins unter vermindertem Druck wird der feste Rückstand mit Diäthyläther gewaschen. Man erhält 2',5'-Bis-(5-di-N,N-n-butylcarbamoyl-2-methyl-pent-2-yl)-hydrochinon vom Smp. 175—9° C. Nach zwei weiteren Kristallisationen aus Äthanol erhält man ein Produkt vom Smp. 190—2° C.

Analyse für $C_{36}H_{64}N_2O_4$:

| | | | |
|---|---|---|---|
| gefunden | C 73,06 | H 11,35 | N 4,83%; |
| berechnet | C 73,42 | H 10,95 | N 4,76%. |

Beispiel 25

5,5 Teile Hydrochinon, 19,8 Teile Citronellylacetat und 1,0 Teil p-Toluolsulfonsäure werden 24 Stunden auf einem Dampfbad erhitzt. Dann wird das Reaktionsgemisch in Diäthyläther aufgenommen, mit 10%iger Natriumhydroxidlösung und Wasser gewaschen und verdampft. Der erhaltene Rückstand wird bei 0,13 Millibar mittels Kurzwegdestillation destilliert und ergibt 2',5'-Bis-(8-acetoxy-2,6-dimethyl-oct-2-yl)-hydrochinon in Form eines viskosen bernsteinfarbenem Öls, das sich langsam verfestigt. Durch Kristallisation aus Petroläther mit einem Siedebereich von 60—80° C erhält man ein Produkt vom Smp. 60—2° C.

Analyse für $C_{36}H_{62}O_{6}$:

gefunden C 73,18 H 10,58%;
berechnet C 73,15 H 10,76%.

Beispiel 26

5,5 Teile Hydrochinon, 15,4 Teile 1-Methyl-4-methoxycarbonylcyclohex-1-en und 0,5 Teile p-Toluolsulfonsäure werden drei Tage auf einem Dampfbad erhitzt. Nach dem Verdünnen des Reaktionsgemisches mit Diäthyläther erhält man ein Stereoisomerengemisch von 2',5'-Bis-(4-methoxycarbonyl-1-methyl-cyclohex-1-yl)-hydrochinon vom Smp. 266—83° C. Durch Kristallisation aus Dimethylformamid und Wasser erhält man ein Produkt mit einem Schmelzpunkt von 268—87° C, das 1 Mol kristallisiertes Dimethylformamid enthält.

Analyse für $C_{24}H_{34}O_{6} \cdot C_{3}H_{7}NO$:

gefunden C 65,65 H 8,32 N 2,28%;
berechnet C 65,96 H 8,41 N 2,85%.

Beispiel 27

12,4 Teile Toluylhydrochinon, 21,2 Teile 5-Methyl-hex-5-ensäure-n-hexylester und 0,3 Teile p-Toluolsulfonsäure werden wie in Beispiel 25 beschrieben umgesetzt und aufgearbeitet. Die Destillation unter vermindertem Druck ergibt eine bei 217—26° C/0,1 bar siedende Fraktion, die gemäß Analyse aus 14% n-Hexyl-5-(2',5'-dihydroxy-3'-methylphenyl)-5-methylhexanoat und 82% n-Hexyl-5-(2',5'-dihydroxy-4'-methylphenyl)-5-methylhexanoat besteht.

Analyse für $C_{20}H_{32}O_{4}$:

gefunden C 71,24 H 10,11%;
berechnet C 71,39 H 9,59%.

Beispiel 28

16,6 Teile 2-tert.-Butylhydrochinon, 14,2 Teile 5-Methyl-hex-5-ensäuremethylester und 0,5 Teile p-Toluolsulfonsäure werden wie in Beispiel 25 beschrieben umgesetzt und aufgearbeitet. Nach der Destillation erhält man Methyl-5-(4'-tert.-butyl-2',5'-dihydroxyphenyl)-5-methyl-hexanoat; Sdp. 186—210° C/0,3 bar.

Nach der Kristallisation aus Petroläther mit einem Siedebereich von 40—60° C hat diese Fraktion (dieses Produkt) einem Smp. von 136—9° C.

Analyse für $C_{18}H_{28}O_{4}$:

gefunden C 70,39 H 9,39%;
berechnet C 70,10 H 9,15%.

Beispiel 29

5,0 Teile des gemäß Beispiel 28 erhaltenen Esters werden mit 20 Teilen n-Hexanol und 0,25 Teilen Natriummethoxid in einem zugeschmolzenen Glasrohr während 7 Tagen zum entsprechenden n-Hexylester umgesetzt. Das Reaktionsgemisch wird mit Diäthyläther verdünnt, mit verdünnter HCl und dann mit Wasser gewaschen und unter vermindertem Druck von Diäthyläther und überschüssigem n-Hexanol befreit. Durch Kurzwegdestillation des erhaltenen Rückstandes bei 0,4 mBar erhält man n-Hexyl-5-(4'-tert.-butyl-2',5'-dihydroxyphenyl)-5-methylhexanoat in Form eines bernsteinfarbenen Öls.

Analyse für $C_{23}H_{38}O_{4}$:

gefunden C 72,33 H 10,26%;
berechnet C 72,98 H 10,12%.

## Beispiel 30

Analog Beispiel 29 wird aus n-Decanol und dem gemäß Beispiel 28 erhaltenen Ester das n-Dodecyl-5-(4'-tert.-butyl-2',5'-dihydroxyphenyl)-5-hexanoat hergestellt.

Analyse für $C_{29}H_{50}O_4$:

gefunden C 75,49 H 11,01%;
berechnet C 75,28 H 10,89%.

## Beispiel 31

83 Teile 2-tert.-Butylhydrochinon, 17,8 Teile Dimethylprenylphosphonat 0,5 Teile Fulmont® 327 werden 24 Stunden bei 130° C gerührt. Das abgekühlte Reaktionsgemisch wird mit Diäthyläther verdünnt und durch Filtrieren vom Katalysator befreit. Die ätherische Lösung wird zuerst mit 10%iger Natriumhydroxidlösung gewaschen, um restliches tert.-Butylhydrochinon zu entfernen, und dann mit Wasser. Der Ätherextrakt wird anschließend verdampft, und der Rückstand mit Petroläther, der Diäthyläther enthält und einen Siedebereich von 40—60° C aufweist, verdünnt. Nach dem Stehenlassen bei 0° C erhält man einen hellbraunen Farbstoff, Smp. 153—65° C. Nach einer weiteren Kristallisation mit Diäthyläther, der etwas Aceton enthält, erhält man das Dimethyl-3-(4'-tert.-butyl-2',5'-dihydroxyphenyl)-3-methylbutanphosphonat; Smp. 193—5° C.

Analyse für $C_{17}H_{29}O_5P$:

gefunden C 59,99 H 8,38 P 8,62%;
berechnet C 59,29 H 8,49 P 8,99%.

## Beispiel 32

83 Teile tert.-Butylhydrochinon, 19,8 Teile 5,7,7-Trimethyl-oct-4-ensäuremethylester und 5,0 Teile Fulmont® 237 werden wie in Beispiel 31 beschrieben umgesetzt und aufgearbeitet. Das Produkt wird bei 0,13 Millibar einer Kurzweg-Rotationsdestillation unterworfen. Nach dem Entfernen von niedrigsiedenden Substanzen erhält man Methyl-5-(4'-tert.-butyl-2',5'-dihydroxyphenyl)-5,7,7-trimethyloctanoat.

Analyse für $C_{22}H_{36}O_4$:

gefunden C 72,72 H 9,32%;
berechnet C 72,49 H 9,95%.

## Beispiel 33

Die bei der Herstellung von 2',5'-Bis-(5-methoxycarbonyl-2-methylpent-2-yl)-hydrochinon gemäß Beispiel 1 erhaltene Mutterlauge wird konzentriert und dann destilliert. Eine bei 160—240° C/0,5 bar erhaltene Fraktion wird mit Petroläther (Siedebereich 40—60° C) verdünnt. Aus dieser Lösung kristallisiert das 2'-(5-Methoxycarbonyl-2-methylpent-2-yl)-hydrochinon aus; Smp. 102—4° C.

Analyse für $C_{14}H_{20}O_4$:

gefunden C 66,65 H 7,99%;
berechnet C 66,45 H 8,45%.

## Beispiel 34

5,5 Teile Hydrochinon, 22,8 Teile 1,7-Dimethoxycarbonyl-4-methyl-hept-3-en und 1,0 Teile p-Toluolsulfonsäure werden drei Tage auf einem Dampfbad erhitzt. Nach dem Verdünnen mit Diäthyläther wird das Reaktionsgemisch mit Natriumbicarbonatlösung und mit Wasser gewaschen, verdampft und destilliert. Man erhält Dimethyl-5-methyl-5-(2',5'-dihydroxyphenyl)-azelat; Sdp. 260—9° C/0,1 bar.

Analyse für $C_{18}H_{26}O_6$:

gefunden C 64,34 H 7,92%;
berechnet C 63,89 H 7,74%.

Beispiel 35

41,4 Teile 2-tert.-Butylhydrochinon, 15,3 Teile Diäthyl-2-äthoxycarbonyl-5-methyl-hex-4-en-2-phosphonat und 5,0 Teile Fulmont® 237 werden 24 Stunden bei 130° C gerührt. Das abgekühlte Reaktionsgemisch wird mit Diäthyläther verdünnt, mit 10%iger Natriumhydroxidlösung und dann mit Wasser gewaschen. Nach dem Abziehen des Diäthyläthers wird das zurückgebliebene Öl destilliert. Man erhält eine bei 89–156° C/0,4 bar siedende Fraktion und einen Rückstand. Dieser Rückstand wird an einer aus 150 Teilen Kieselsäure und einem anfänglichen Lösungsmittelgemisch aus Petroläther (Siedebereich 40–60° C) und 5% Äthylacetat bestehenden Säule chromatographiert. Das Eluieren der Säule erfolgt durch Erhöhen des Äthergehaltes, bis schließlich zu einem Äthergehalt von 50%. Man erhält Diäthyl-5-(4'-tert.-butyl-2',5'-dihydroxyphenyl)-2-äthoxycarbonyl-5-methyl-hexan-2-phosphonat in Form eines viskosen Öls.

Analyse für $C_{24}H_{41}O_7P$:

| | | | |
|---|---|---|---|
| gefunden | C 61,16 | H 8,52 | P 6,61%; |
| berechnet | C 60,99 | H 8,74 | P 6,55%. |

Beispiel 36

2,5 Teile Hydrochinon, 8,5 Teile n-Butylcitronellyläther und 0,5 Teile p-Toluolsulfonsäure werden 3 Tage auf einem Dampfbad erhitzt. Die Aufarbeitung erfolgt wie in Beispiel 25 angegeben mittels Kurzwegdestillation bei 0,5 Millibar. Man erhält 2',5'-Bis-(8-n-butoxy-2,6-dimethyl-oct-2-yl)-hydrochinon vom Smp. 86–9° C.

Analyse für $C_{34}H_{62}O_4$:

| | | |
|---|---|---|
| gefunden | C 76,43 | H 11,88%; |
| berechnet | C 76,35 | H 11,68%. |

Beispiel 37

Analog der Arbeitsweise gemäß Beispiel 36 erhält man unter Verwendung von 6,2 Teilen Citronellol anstelle des n-Butylcitronellyläthers 2',5'-Bis-(2,6-dimethyl-8-hydroxy-oct-2-yl)-hydrochinon in Form eines viskosen Öls.

Analyse für $C_{26}H_{46}O_4$:

| | | |
|---|---|---|
| gefunden | C 74,18 | H 11,07%; |
| berechnet | C 73,89 | H 10,97%. |

Beispiel 38

Nach dem Verfahren gemäß Beispiel 2 werden 4,8 Teile 2',5'-Bis-(5-carboxy-2-methyl-pent-2-yl)-hydrochinon, 50 Teile Tetrahydropyran-2-methanol und 1,0 Teil p-Toluolsulfonsäure zur Herstellung von 2',5'-Bis-(5-tetrahydropyran-4-yloxycarbonyl-2-methyl-pent-2-yl)-hydrochinon, Smp. 155–8° C, verwendet.

Analyse für $C_{32}H_{50}O_8$:

| | | |
|---|---|---|
| gefunden | C 68,62 | H 9,05%; |
| berechnet | C 68,30 | H 8,96%. |

Beispiel 39

5,0 Teile 2',5'-Bis-(5-carboxy-2-methyl-pent-2-yl)-hydrochinon, 50 Teile eines handelsüblichen Iso-octanols (Isomerengemisch, vertrieben durch ICI Ltd.) und 1,0 Teil p-Toluolsulfonsäure werden nach dem in Beispiel 2 beschriebenen Verfahren umgesetzt und aufgearbeitet. Kurzwegdestillation bei 0,5 Millibar und einer Ofentemperatur von 240° C ergibt 2',5'-Bis-(2-methyl-5-iso-octyloxycarbonyl-pent-2-yl)-hydrochinon in Form eines viskosen Öls.

Analyse für $C_{36}H_{62}O_6$:

| | | |
|---|---|---|
| gefunden | C 73,94 | H 10,87%; |
| berechnet | C 73,18 | H 10,58%. |

Beispiel 40

1,1 Teile Hydrochinon, 4,5 Teile Äthyl-2-äthoxycarbonyl-5-methyl-hex-4-enoat und 0,5 Teile p-Toluolsulfonsäure werden drei Tage auf einem Dampfbad erhitzt. Dann wird das Reaktionsgemisch mit Diäthyläther verdünnt, mit 10%iger Natriumhydroxidlösung und Wasser gewaschen und verdampft. Durch Kurzwegdestillation bei 0,7 Millibar und einer Ofentemperatur von 120° C werden niedrig-siedende Substanzen entfernt. Der Rückstand wird aus Petroläther (Siedebereich 40–60° C), der 5% Äthylacetat enthält, kristallisiert. Man erhält 2',5'-Bis-(5,5-diäthoxycarbonyl-2-methyl-pent-2-yl)-hydrochinon; Smp. 157–60° C.

Analyse für $C_{30}H_{45}O_{10}$:

| | | |
|---|---|---|
| gefunden | C 63,90 | H 8,28%; |
| berechnet | C 63,59 | H 8,18%. |

Verwendungsbeispiele (Verwendung der neuen Hydrochinone der Formel I als Stabilisatoren in Farbphotographiematerialien)

Verwendungsbeispiel 1

0,05 mMol des Magenta-Kupplers der Formel

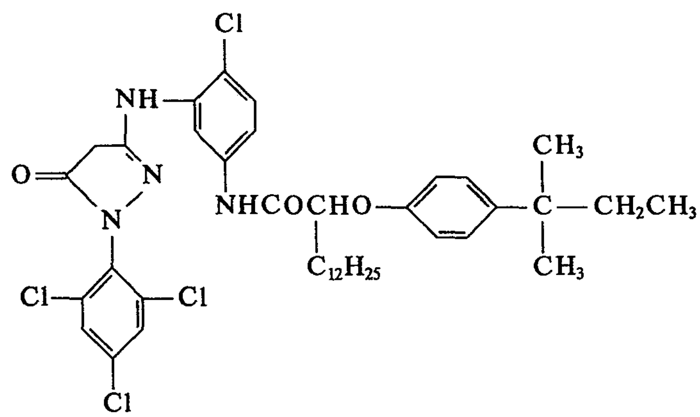

und 0,025 mMol einer Verbindung der Formel I werden in 2,0 ml Trikresylphosphat/Äthylacetat (0,75 g/100 ml) gelöst. 7,0 ml einer 6%igen Gelatinelösung, 1,0 ml einer 0,8%igen Lösung des Netzmittels der Formel

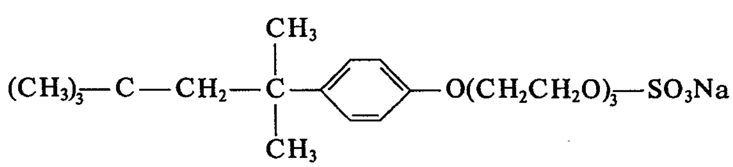

werden in Wasser eingetragen, dann 5 Minuten mit Hilfe eines 100 Watt-Ultraschallgeräts emulgiert. 2,5 ml der frisch im Ultraschallgerät behandelten Kuppler-Additiv-Emulsion, 2,0 ml einer Silberbromidemulsion mit einem Silbergehalt von 0,6%, 0,7 ml einer 1%igen wäßrigen Lösung des Härters der Formel

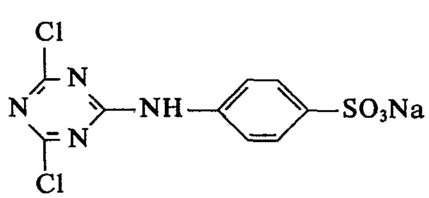

und 2,8 ml Wasser werden gemischt, auf eine pH-Wert von 6,5 eingestellt und bei 40° C auf ein Polyäthylenpapier mit den Maßen 14 × 18 cm gegossen. Nach dem Härten der Schicht bei 10° C wird das aufgegossene Gemisch bei Raumtemperatur getrocknet.

Verarbeitung

Muster des erhaltenen beschichteten Papiers werden 6 Sekunden unter einem Stufenkeil mit Licht von 600 Lux bestrahlt und dann wie folgt bei 32,8° C (+0,3° C) behandelt:

| | |
|---|---|
| 1. Entwicklerbad | 3,5 Minuten |
| 2. Bleich- und Fixierbad | 1,5 Minuten |
| 3. Waschen | 3,0 Minuten |
| 4. Trocknen | 1,0 Minuten |

Das Entwicklerbad hat die folgende Zusammensetzung:

4-Amino-3-methyl-N-äthyl-N-[β-(methyl-sulfonamido)äthyl]-anilin

| | |
|---|---|
| $1^1/_2\ H_2SO_4 \cdot H_2O$ | 4,85 (g/Liter) |
| Kaliumbromid | 0,6 |
| Kaliumcarbonat | 32,0 |
| Lithiumsulfat | 1,8 |
| Kaliumsulfit | 2,0 |
| Hydroxylaminsulfit | 3,9 |
| Äthylenglykol | 21,3 |
| Benzylalkohol | 15,1 |
| Wasser | bis zu einem Liter |

Der pH-Wert beträgt 10,1.

Als Bleich- und Fixierbad wird ein übliches Bad eingesetzt, z. B. mit der folgenden Zusammensetzung:

| | |
|---|---|
| Ammoniumthiosulfat (80%ige Lösung) | 200 (g/Liter) |
| Natriumsulfit (wasserfrei) | 15 |
| Natriumcarbonat (wasserfrei) | 2,5 |
| Äthylendiamin-tetraessigsäure-Na-Salz | 2 |
| Na-Salz von Fe(III)-Äthylendiamin-tetraessigsäure | 30 |
| Wasser | bis zu einem Liter |

Nach dem Waschen und Trocknen erhält man klare, scharfe Magenta-Keile mit einem Absorptionsmaximum bei 537 nm und maximaler Dichte von 2.28. Ein auf diese Weise erhaltener Stufenkeil wird in einem Atlas-Gerät (2500 W-Lampe) durch ein Ultraviolett-Filter (Kodakfilter 2C) mit 42 $kJ/cm^2$ bestrahlt. Als Vergleich dient ein Stufenkeil, der ohne Zusatz einer erfindungsgemäßen Verbindung hergestellt wurde. Die restliche optische Dichte (OD) wird jeweils in % der Anfangsdichte (Anfangsdichte 1) gemessen. Die Ergebnisse sind in Tabelle II zusammengefaßt.

Tabelle II
Lichtstabilisierende Wirkung von Verbindungen der Formel I

| Hydrochinon | % OD (mit UV-Filter; 42 kJ/cm$^2$) |
|---|---|
| Ohne Stabilisator | 37 |
| Verbindung gemäß Beispiel 9 | 80 |
| Verbindung gemäß Beispiel 11 | 84 |
| Verbindung gemäß Beispiel 12 | 87 |
| Verbindung gemäß Beispiel 14 | 81 |
| Verbindung gemäß Beispiel 15 | 85 |
| Verbindung gemäß Beispiel 18 | 78 |
| Verbindung gemäß Beispiel 25 | 76 |
| Ohne Stabilisator | 41 |
| Verbindung gemäß Beispiel 19 | 85 |
| Verbindung gemäß Beispiel 17 | 88 |
| Verbindung gemäß Beispiel 29 | 87 |
| Verbindung gemäß Beispiel 30 | 91 |
| Verbindung gemäß Beispiel 32 | 78 |
| Verbindung gemäß Beispiel 21 | 58 |
| Verbindung gemäß Beispiel 24 | 55 |

Im Vergleich zu der ohne Stabilisator hergestellten Emulsion zeigen die Verbindungen der Formel I enthaltenden Emulsionen eine bessere Lichtschutzwirkung.

Verwendungsbeispiel 2

Es werden Emulsionen nach dem in Verwendungsbeispiel 1 beschriebenen Verfahren zubereitet, die jedoch anstelle des dort genannten Magenta-Kupplers 0,05 mMol des Magenta-Kupplers der Formel

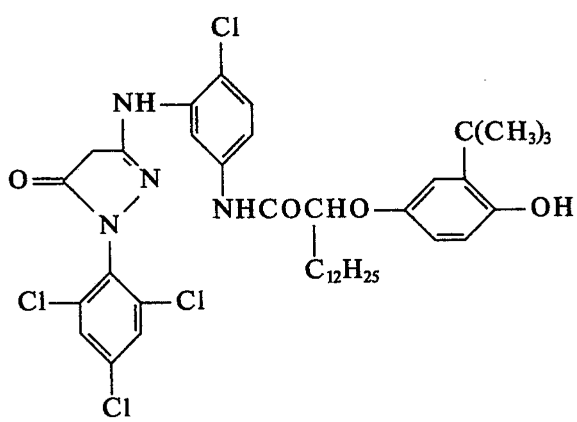

sowie 0,025 mMol der Verbindung gemäß Beispiel 9 enthalten. Die Emulsionen werden im Atlas-Gerät auf analoge Weise mit 42 kJ/cm$^2$ bestrahlt. Die Zunahme der Dichte wird bei einer Wellenlänge von 436 nm (Vergilbung) gemessen. Es werden die folgenden Resultate erhalten:

Tabelle III

Lichtschutzwirkung der Verbindungen gemäß Beispiel 9

| Hydrochinon | Zunahme 100 $D_{436}$ nach 42 kJ/cm$^2$ im Atlas-Gerät |
|---|---|
| Ohne Stabilisator | 10 |
| Verbindung gemäß Beispiel 9 | −1 |

Verglichen mit der keinen Stabilisator enthaltenden Emulsion zeigt die die Verbindung gemäß Beispiel 9 enthaltende Emulsion keine Zunahme der Dichte bei 436 nm.

Beispiel 41

5,0 Teile 2',5'-Bis-(5-carboxy-2-methyl-pent-2-yl)-hydrochinon, 50 Teile einer Mischung Amyl-/iso-Butylalkohol (vertrieben durch Hoechst AG) und 0,5 Teile Toluolsulfonsäure werden nach dem in Beispiel 2 beschriebenen Verfahren umgesetzt und aufgearbeitet. Nach dem Einengen der ätherischen Lösung und nach dem Verdünnen mit Petroläther (Siedebereich 6 60—80° C) erhält man eine Amyl- und iso-Butylestermischung des Ausgangsproduktes vom Smp. 106—20° C.

Analyse für $C_{29}H_{48}O_6$:

gefunden C 70,82 H 9,82%;
berechnet C 70,70 H 9,82%.

Beispiel 42

Analog Beispiel 39 werden 5,0 Teile 2',5'-Bis-(5-carboxy-2-methylpent-2-yl)-hydrochinon mit 50 Teilen Epal 108 (n-Decanol-Octanol-Mischung, vertrieben durch Ethyl Corporation) verestert. Nach Kurzwegdestillation wird der entsprechende Ester in Form eines viskosen Öles erhalten.

Analyse für $C_{38}H_{66}O_6$:

gefunden C 74,49 H 11,00%;
berechnet C 73,74 H 10,75%.

Verwendungsbeispiel 4

Auf analoge Weise wie im Verwendungsbeispiel 1 beschriebenen Verfahren werden weitere Verbindungen der Formel I geprüft. Die Ergebnisse sind in der Tabelle IV zusammengefaßt.

Tabelle IV

| Hydrochinon | % OD (mit UV-Filter; 42 kJ/cm$^2$) |
|---|---|
| Ohne Lichtstabilisator | 45 |
| Verbindung gemäß Beispiel 16 | 87 |
| Verbindung gemäß Beispiel 27 | 80 |
| Verbindung gemäß Beispiel 41 | 90 |
| Verbindung gemäß Beispiel 42 | 90 |

Tabelle IV

| Hydrochinon | % OD (mit UV-Filter; 21 $kJ/cm^2$) |
|---|---|
| Ohne Lichtstabilisator | 84 |
| Verbindung gemäß Beispiel 35 | 94 |
| Verbindung gemäß Beispiel 36 | 97 |
| Verbindung gemäß Beispiel 37 | 93 |
| Verbindung gemäß Beispiel 38 | 96 |
| Verbindung gemäß Beispiel 39 | 98 |
| Verbindung gemäß Beispiel 40 | 96 |
| Verbindung gemäß Beispiel 41 | 98 |

**Patentansprüche**

1. Hydrochinone der Formel I

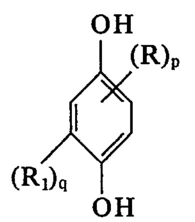

(I)

worin p 1 oder 2 und q 0 oder 1 sind, mit der Maßgabe, daß p + q 1 oder 2 sind,

R einen Rest der Formel II

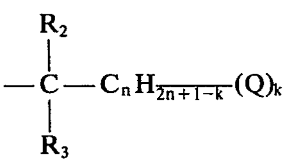

(II)

Q $-COOR_4$, $-CONR_4R_5$, $-OR_5$, $-OCOR_7$, $-NR_8R_9$, $-PO(OR_{10})([O]_kR_{11})$, $-SO_2OH$, $-SO_2Cl$, $-SO_2NR_5R_7$ oder $-CN$ bedeuten,
n eine ganze Zahl von 1 bis 20 und
k eine ganze Zahl 1 oder 2 bedeuten, und
$R_1$ $C_{1-8}$-Alkyl oder einen Rest der Formel II bedeutet, wobei R und $R_1$ gleiche oder verschiedene Reste der Formel II darstellen können,
$R_2$ und $R_3$ unabhängig voneinander $C_{1-5}$-Alkyl bedeuten und, wenn Q $-COOR_4$ ist, $R_2$ oder $R_3$ durch eine Gruppe $-COOR_4$ substituiert sein können, oder $R_2$ oder $R_3$ so an den Rest $-C_nH_{2n+1-k}$ gebunden sind, daß eine durch Gruppen $-(COOR_4)_k$ substituierte $C_{5-12}$-Cycloalkylengruppe gebildet wird, wobei die $R_4$ gleiche oder verschiedene Bedeutungen haben können,
$R_4$ Wasserstoff, $C_{1-20}$-Alkyl, das durch 1–5 Sauerstoffatome unterbrochen und/oder durch eine Gruppe $-OR_6$ substituiert sein kann, wobei $R_6$ $C_{1-12}$-Alkyl, $C_{3-12}$-Cycloalkyl, $C_{3-20}$-Alkenyl, $C_{7-13}$-Aralkyl oder $C_{6-10}$-Aryl bedeutet, wobei die Arylgruppen durch eine oder zwei $C_{1-4}$-Alkylgruppen substituiert sein kann, ferner $R_4$ $C_{3-20}$-Alkenyl, $C_{3-12}$-Cycloalkyl, $C_{7-13}$-Aralkyl, $C_{6-10}$-Aryl, das durch eine $C_{1-4}$-Alkylgruppe substituiert sein kann, einen 5- oder 6-gliedrigen O-haltigen Heterocyclus, der durch eine oder zwei $C_{1-4}$-Alkylgruppen substituiert sein kann, oder Methyl, das durch einen sauerstoffhaltigen 5- oder 6-gliedrigen heterocyclischen Ring substituiert ist,
$R_5$ Wasserstoff oder $C_{1-20}$-Alkyl bedeuten oder
$R_4$ und $R_5$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,

$R_7$ Wasserstoff, $C_{1-20}$-Alkyl, $C_{3-20}$-Alkylen, $C_{3-12}$-Cycloalkyl, $C_{7-13}$-Aralkyl oder $C_{6-10}$-Aryl, das durch eine oder zwei $C_{1-4}$-Alkylgruppen substituiert sein kann, bedeutet,
$R_8$ Wasserstoff oder $C_{1-12}$-Alkyl und
$R_9$ Wasserstoff, $C_{1-12}$-Alkyl oder eine Gruppe $-COR_7$ darstellen oder
$R_8$ und $R_9$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann,
x 0 oder 1,
$R_{10}$ und $R_{11}$ bei $x = 1$ unabhängig voneinander Wasserstoff oder $C_{1-20}$-Alkyl bedeuten oder $R_{10}$ und $R_{11}$ zusammen $C_{2-3}$-Alkylen bilden, das durch 1 oder mehrere $C_{1-20}$-Alkylgruppen substituiert sein kann, und bei $x = 0$
$R_{10}$ Wasserstoff oder $C_{1-20}$-Alkyl, und
$R_{11}$ $C_{1-5}$-Alkyl darstellen,

mit der Maßgabe, daß $R_1$ bei $Q = -SO_2OH$ einen Rest der Formel II darstellt.

2. Verbindungen der Formel I nach Anspruch 1, worin p, q, R, $R_1$, $R_2$, $R_3$, $R_4$, $R_5$, $R_7$, Q, n und k die im Anspruch 1 angegebene Bedeutung haben, $R_6$ $C_{3-12}$-Cycloalkyl, $C_{3-20}$-Alkenyl, $C_{7-13}$-Aralkyl oder $C_{6-10}$-Aryl bedeutet, wobei die Arylgruppe durch eine oder zwei $C_{1-4}$-Alkylgruppen substituiert sein kann, $R_8$ Wasserstoff oder $C_{1-4}$-Alkyl und $R_9$ Wasserstoff, $C_{1-4}$-Alkyl oder eine Gruppe $-COR_7$ darstellen oder $R_8$ und $R_9$ zusammen mit dem Stickstoffatom, an das sie gebunden sind, einen 5- oder 6-gliedrigen Heterocyclus bilden, der durch eine oder zwei $C_{1-4}$-Alkylgruppen substituiert sein kann, und $R_{10}$ und $R_{11}$ bei $x = 1$ unabhängig voneinander Wasserstoff oder $C_{1-20}$-Alkyl bedeuten oder $R_{10}$ und $R_{11}$ zusammen $C_{2-3}$-Alkylen bilden, das durch 1 oder mehrere $C_{1-20}$-Alkylgruppen substituiert sein kann, und $R_{10}$ bei $x = 0$, n-Alkyl mit 1–5 C-Atomen darstellt.

3. Verbindungen der Formel I nach Anspruch 1, worin die Gruppen R und $R_1$ in 2-Stellung resp. 5-Stellung des Hydrochinonkerns stehen.

4. Verbindungen der Formel III nach Anspruch 1

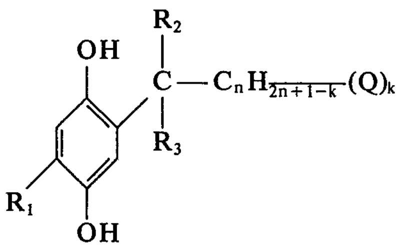

(III)

worin $R_2$, $R_3$, n, k und Q die im Anspruch 1 angegebene Bedeutung haben, und $R_1$ eine Gruppe der Formel II gemäß der im Anspruch 1 angegebenen Definition, oder eine Gruppe der Formel IV

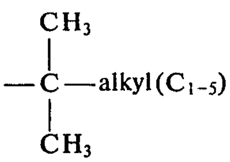

(IV)

sowie Salze davon.

5. Verbindungen der Formel III nach Anspruch 4, worin $R_1$ die im Anspruch 4 angegebene Bedeutung hat, n und k die im Anspruch 1 angegebene Bedeutung haben, Q $-COOR_4$, $-CONR_4R_5$ oder $-OR_5$ bedeutet, wobei $R_4$ und $R_5$ die im Anspruch 1 angegebene Bedeutung haben, $R_2$ und $R_3$ unabhängig voneinander Methyl, Äthyl, n-Propyl, Isopropyl oder Neopentyl bedeuten, oder entweder $R_2$ oder $R_3$ durch eine Gruppe $-COOR_4$ substituiert sein können oder $R_2$ oder $R_3$ so an den Rest $-C_nH_{2n+1-k}$ gebunden sind, daß ein durch $-COOR_4$ substituierter $C_{5-8}$-Cycloalkylenrest gebildet wird, sowie Salze dieser Verbindungen.

6. Verbindungen der Formel III nach Anspruch 5, worin k die Zahl 1 ist, $R_1$ die im Anspruch 4 angegebene Bedeutung hat, Q $-COOR_4$, $-CONR_24R_5$ oder $-OR_5$ darstellt, n eine Zahl von 1 bis 10 ist, $R_2$ und $R_3$ unabhängig voneinander Methyl, Äthyl oder Neopentyl darstellen, oder entweder $R_2$ oder $R_3$ so an den Rest $C_nH_{2n+1-k}$ gebunden sind, daß ein durch $-COOR_4$ substituierter Cyclohexylrest gebildet wird, $R_4$ Wasserstoff oder $C_{1-20}$-Alkyl bedeutet, das durch 1 bis 3 Sauerstoffatome unterbrochen und/oder durch eine Gruppe $-OR_6$ substituiert sein kann, wobei $R_6$ Cyclopentyl, Cyclohexyl, Cyclooctyl, $C_{3-10}$-Alkenyl, Phenyl, Benzyl, Phenäthyl, Benzhydryl oder Naphthylmethyl bedeutet, ferner $R_4$ $C_{3-15}$-Alkenyl, Phenyl, das durch 1 oder zwei $C_{1-4}$-Alkylgruppen substituiert sein kann, Benzyl, Phenäthyl, Cyclopentyl, Cyclohexyl oder einen sauerstoffhaltigen 5- oder 6-gliedrigen heterocyclischen Ring bedeutet, der durch eine oder zwei $C_{1-4}$-Alkylgruppen substituiert sein kann, und $R_5$ die im Anspruch 1 angegebene

Bedeutung hat, oder $R_4$ und $R_5$ zusammen mit dem Stickstoffatom, an das sie gebunden sind, einen 5- oder 6-gliedrigen heterocyclischen Ring bilden können, der durch eine oder zwei $C_{1-4}$-Alkylgruppen substituiert sein kann, sowie Salze dieser Verbindungen.

7. Verbindungen der Formel III nach Anspruch 6, worin $R_1$, k, n, $R_2$ und $R_3$ die im Anspruch 6 angegebene Bedeutung haben, Q $-COOR_4$, $-CONR_4R_5$ oder $-OR_5$ darstellt, $R_4$ Wasserstoff, $C_{1-20}$-Alkyl, das durch 1 oder 2 Sauerstoffatome unterbrochen und/oder durch Cyclohexyloxy, $C_{3-10}$-Alkenyloxy, Phenoxy oder Benzyloxy substituiert sein kann, ferner $R_4$ $C_{3-15}$-Alkenyl, Phenyl, Benzyl, Phenäthyl, Cyclopentyl, Cyclohexyl, einen sauerstoffhaltigen 5- oder 6-gliedrigen heterocyclischen Ring, und $R_5$ Wasserstoff oder $C_{1-15}$-Alkyl bedeutet, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann.

8. Verbindungen der Formel III nach Anspruch 7, worin k, $R_1$, Q, n, $R_2$, $R_3$ und $R_5$ die im Anspruch 7 angegebene Bedeutung haben, und $R_4$ Wasserstoff oder $C_{1-16}$-Alkyl, das durch ein Sauerstoffatom unterbrochen und/oder durch Phenoxy substituiert sein kann, ferner $R_4$ $C_{3-15}$-Alkenyl, Phenyl, Benzyl, Tetrahydrofuran-3-yl, Tetrahydropyran-4-yl oder Tetrahydrofurfuryl bedeutet, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann, sowie Salze davon.

9. Verbindungen der Formel III nach Anspruch 8, worin k die Zahl 1, $R_1$ die im Anspruch 4 angegebene Bedeutung hat, Q $-COOR_4$, $-CONR_4R_5$ oder $-OR_5$ darstellt, n die Zahl 1 bis 3 ist, $R_2$ und $R_3$ Methyl oder Neopentyl darstellen, $R_4$ Wasserstoff, $C_{1-16}$-Alkyl, das durch ein Sauerstoffatom unterbrochen oder durch Phenoxy substituiert sein kann, ferner $R_4$ Phenyl, Benzyl oder Tetrahydrofuran-3-yl bedeutet, $R_5$ Wasserstoff oder $C_{1-15}$-Alkyl ist, oder $R_4$ und $R_5$ zusammen mit dem Stickstoffatom, an das sie gebunden sind, einen 5- oder 6-gliedrigen heterocyclischen Ring bilden, der durch eine oder zwei $C_{1-4}$-Alkylgruppen substituiert sein kann, mit der Maßgabe, daß wenn Q $-OR_5$ darstellt, $R_5$ $C_{1-8}$-Alkyl bedeutet, sowie Salze davon.

10. Verfahren zur Herstellung von Verbindungen der Formel I nach Anspruch 1, dadurch gekennzeichnet, daß man in Gegenwart eines sauren Katalysators ein Hydrochinon der Formel V

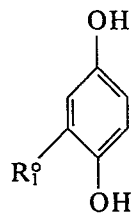

(V)

worin $R_1^o$ Wasserstoff oder die im Anspruch 1 angegebene Bedeutung hat, mit einem zur Einführung einer Gruppe der Formel II befähigten funktionelle Alkylierungsmittel umsetzt, wobei die Formel II die im Anspruch 1 angegebene Bedeutung hat.

11. Verfahren gemäß Anspruch 10, dadurch gekennzeichnet, daß eine Verbindung der Formel I, worin q die Zahl 0 oder 1 und p die Zahl 1 sind, durch Reaktion einer Verbindung der Formel V mit 0,1 bis zu 1,0 Mol des Alkylierungsmittels pro Mol der Verbindung der Formel V hergestellt wird, worin in der Formel V $R_1^o$ Wasserstoff, $C_{1-8}$-Alkyl oder einen Rest der Formel II darstellt.

12. Verfahren gemäß Anspruch 10, dadurch gekennzeichnet, daß Verbindungen der Formel I, worin q die Zahl 0 und p die Zahl 2 darstellen und beide einzuführenden Gruppen R der Formel II gleich sind, durch Reaktion einer Verbindung der Formel V mit mindestens 2 Mol eines Alkylierungsmittels pro Mol der Verbindung der Formel V, hergestellt werden, wobei in der Formel V $R_1^o$ Wasserstoff ist.

13. Verfahren zur Herstellung eines Hydrochinons der Formel I, worin q die Zahl 1 und $R_1$ $C_{4-8}$-Alkyl darstellt, dadurch gekennzeichnet, daß eine Verbindung der Formel I, worin q die Zahl 0 und p die Zahl 1 darstellen, mit mindestens einem Mol $C_{4-8}$-Olefin oder -Alkohol in Gegenwart eines sauren Katalysators umgesetzt wird.

14. Verfahren nach Anspruch 10, dadurch gekennzeichnet, daß das Alkylierungsmittel ein aktives Zentrum enthält, das während der Alkylierungsreaktion eliminiert, verändert oder umgelagert wird.

**Claims**

1. Hydroquinones of the formula I

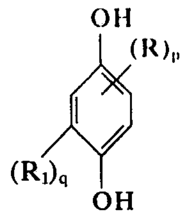

(I)

wherein p is 1 or 2 and Q is 0 or 1, with the proviso that p + q is 1 or 2,

R is a radical of the formula II

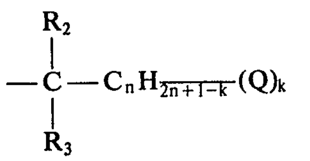

(II)

Q is $-COOR_4$, $-CONR_4R_5$, $-OR_5$, $-OCOR_7$, $-NR_8R_9$, $-PO(OR_{10})([O]_xR_{11})$, $-SO_2OH$, $-SO_2Cl$, $-SO_2NR_5R_7$ or $-CN$,
n is an integer from 1 to 20 and
k is the integer 1 or 2, and
$R_1$ is $C_1-C_8$-alkyl or a radical of the formula II, and R and $R_1$ can be identical or different radicals of the formula II,
$R_2$ and $R_3$ independently of one another are each $C_1-C_5$-alkyl and, when Q is $-COOR_4$, $R_2$ or $R_3$ can be substituted by a group $-COOR_4$, or $R_2$ or $R_3$ is so bound to the radical $-C_nH_{2n+1-k}$ that a $C_5-C_{12}$-cycloalkylene group substituted by $-(COOR_4)_k$ groups is formed, wherein the $R_4$ symbols can have identical or different meanings,
$R_4$ is hydrogen, $C_1-C_{20}$-alkyl which can be interrupted by 1—5 oxygen atoms and/or substituted by a group $-OR_6$, wherein $R_6$ is $C_1-C_{12}$-alkyl, $C_3-C_{12}$-cycloalkyl, $C_3-C_{20}$-alkenyl, $C_7-C_{13}$-aralkyl or $C_6-C_{10}$-aryl, where the aryl group can be substituted by one or two $C_1-C_4$-alkyl groups, $R_4$ is also $C_3-C_{20}$-alkenyl, $C_3-C_{12}$-cycloalkyl, $C_7-C_{13}$-aralkyl, $C_6-C_{10}$-aryl which can be substituted by a $C_1-C_4$-alkyl group, or is a 5- or 6-membered O-containing heterocycle which can be substituted by one or two $C_1-C_4$-alkyl groups, or is methyl which is substituted by an oxygen-containing 5- or 6-membered heterocyclic ring,
$R_5$ is hydrogen or $C_1-C_{20}$-alkyl, or
$R_4$ and $R_5$ together with the nitrogen atom to which they are attached form a 5- or 6-membered heterocycle which can be substituted by one or two $C_1-C_4$-alkyl groups,
$R_7$ is hydrogen, $C_1-C_{20}$-alkyl, $C_3-C_{20}$-alkenyl, $C_3-C_{12}$-cycloalkyl, $C_7-C_{13}$-aralkyl or $C_6-C_{10}$-aryl, which can be substituted by one or two $C_1-C_4$-alkyl groups,
$R_8$ is hydrogen or $C_1-C_{12}$-alkyl and
$R_9$ is hydrogen, $C_1-C_{12}$-alkyl or a group $-COR_7$, or
$R_8$ and $R_9$ together with the nitrogen atom to which they are attached form a 5- or 6-membered heterocycle which can be substituted by one or two $C_1-C_4$-alkyl groups,
x is 0 or 1,
$R_{10}$ and $R_{11}$, when $x = 1$, independently of one another are hydrogen or $C_1-C_{20}$-alkyl, or $R_{10}$ and $R_{11}$ together form $C_2-C_3$-alkylene which can be substituted by 1 or more $C_1-C_{20}$-alkyl groups, and, when $x = 0$
$R_{10}$ is hydrogen or $C_1-C_{20}$-alkyl, and
$R_{11}$ is $C_1-C_5$-alkyl,

with the proviso that $R_1$, when $Q = -SO_2OH$, is a radical of the formula II.

2. Compounds of the formula I according to claim 1, wherein p, q, R, $R_1$, $R_2$, $R_3$, $R_4$, $R_5$, $R_7$, Q, n and k have the meanings given in claim 1, $R_6$ is $C_3-C_{12}$-cycloalkyl, $C_3-C_{20}$-alkenyl, $C_7-C_{13}$-aralkyl or $C_6-C_{10}$- aryl, the aryl group being able to be substituted by one or two $C_1-C_4$-alkyl groups, $R_8$ is hydrogen or $C_1-C_4$-alkyl and $R_9$ is hydrogen, $C_1-C_4$-alkyl or a group $-COR_7$, or $R_8$ and $R_9$ together with the nitrogen atom to which they are attache form a 5- or 6-membered heterocycle which can be substituted by one or two $C_1-C_4$-alkyl groups, and $R_{10}$ and $R_{11}$, when $x = 1$, independently from one another are hydrogen or $C_1-C_{20}$-alkyl, or $R_{10}$ and $R_{11}$ together form $C_2-C_3$-alkylene which can be substituted by 1 or more $C_1-C_{20}$-alkyl groups, and $R_{10}$, when $x = 0$, is n-alkyl having 1—5 C atoms.

3. Compounds of the formula I according to claim 1, wherein the groups R and $R_1$ are in the 2-

position and 5-position, respectively, of the hydroquinone nucleus.

4. Compounds of the formula III according to claim I

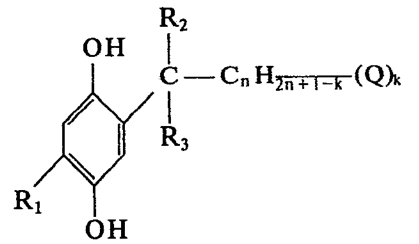

(III)

wherein $R_2$, $R_3$, n, k and Q have the meanings given in claim 1, and $R_1$ is a group of the formula II according to the definition given in claim 1, or a group of the formula IV

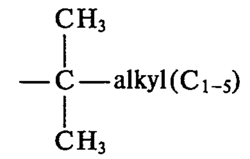

(IV)

and salts thereof.

5. Compounds of the formula III according to claim 4, wherein $R_1$ has the meaning given in claim 4, n and k have the meanings defined in claim 1, Q is $-COOR_4$, $-CONR_4R_5$ or $-OR_5$, wherein $R_4$ and $R_5$ have the meanings given in claim 1, $R_2$ and $R_3$ independently of one another are methyl, ethyl, n-propyl, isopropyl or neopentyl, or either $R_2$ or $R_3$ can be substituted by a group $-COOR_4$, or $R_2$ or $R_3$ are so linked to the radical $-C_nH_{2n+1-k}$ that a $C_5$—$C_8$-cycloalkylene radical substituted by $-COOR_4$ is formed; as well as salts of these compounds.

6. Compounds of the formula III according to claim 5, wherein k is 1, $R_1$ has the meaning defined in claim 4, Q is $-COOR_4$, $-CONR_4R_5$ or $-OR_5$, n is a number from 1 to 10, $R_2$ and $R_3$ independently of one another are methyl, ethyl or neopentyl, or either $R_2$ or $R_3$ is so linked to the radical $C_nH_{2n+1-k}$ that a cyclohexyl group substituted by $-COOR_4$ is formed, $R_4$ is hydrogen or $C_1$—$C_{20}$-alkyl which can be interrupted by 1 to 3 oxygen atoms and/or substituted by a group $-OR_6$, wherein $R_6$ is cyclopentyl, cyclohexyl, cyclooctyl, $C_3$—$C_{10}$-alkenyl, phenyl, benzyl, phenethyl, benzhydryl or naphthylmethyl, or $R_4$ is $C_3$—$C_{15}$-alkenyl, phenyl which can be substituted by 1 or two $C_1$—$C_4$-alkyl groups, or is benzyl, phenethyl, cyclopentyl, cyclohexyl or an oxygen-containing 5- or 6-membered heterocyclic ring which can be substituted by one or two $C_1$—$C_4$-alkyl groups, and $R_5$ has the meaning given in claim 1, or $R_4$ and $R_5$ together with the nitrogen atom to which they are linked can form a 5- or 6-membered heterocyclic ring, which can be substituted by one or two $C_1$—$C_4$-alkyl groups; as well as the salts of these compounds.

7. Compounds or the formula III according to claim 6, wherein $R_1$, k, n, $R_2$ and $R_3$ have the meanings given in claim 6, Q is $-COOR_4$, $-CONR_4R_5$ or $-OR_5$, $R_4$ is hydrogen, $C_1$—$C_{20}$-alkyl which can be interrupted by 1 or 2 oxygen atoms and/or substituted by cyclohexyloxy, $C_3$—$C_{10}$-alkenyloxy, phenoxy or benzyloxy, or $R_4$ is $C_3$—$C_{15}$-alkenyl, phenyl, benzyl, phenethyl, cyclopentyl, cyclohexyl, an oxygen-containing 5- or 6-membered heterocyclic ring, and $R_5$ is hydrogen or $C_1$—$C_{15}$-alkyl, or $R_4$ and $R_5$ together with the nitrogen atom to which they are linked form a 5- or 6-membered heterocyclic ring, which can be substituted by one or two $C_1$—$C_4$-alkyl groups.

8. Compounds of the formula III according to claim 7, wherein k, $R_1$, Q, n, $R_2$, $R_3$ and $R_5$ have the meanings given in claim 7, and $R_4$ is hydrogen or $C_1$—$C_{16}$-alkyl, which can be interrupted by an oxygen atom and/or substituted by phenoxy, or $R_4$ is $C_3$—$C_{15}$-alkenyl, phenyl, benzyl, tetrahydrofuran-3-yl, tetrahydropyran-4-yl or tetrahydrofuryl, or $R_4$ and $R_5$ together with the nitrogen atom to which they are attached for a 5- or 6-membered heterocyclic ring, which can be substituted by one or two $C_1$—$C_4$-alkyl groups; as well as salts thereof.

9. Compounds of the formula III according to claim 8, wherein k is 1, $R_1$ has the meaning given in claim 4, Q is $-COOR_4$, $-CONR_4R_5$ or $-OR_5$, n is 1 to 3, $R_2$ and $R_3$ are methyl or neopentyl, $R_4$ is hydrogen, $C_1$—$C_{16}$-alkyl which can be interrupted by an oxygen atom or substituted by phenoxy, or $R_4$ is phenyl, benzyol or tetrahydrofuran-3-yl, $R_5$ is hydrogen or $C_1$—$C_{15}$-alkyl, or $R_4$ and $R_5$ together with the nitrogen atom to which they are linked form a 5- or 6-membered heterocyclic ring, which can be substituted by one or two $C_1$—$C_4$-alkyl groups, with the proviso that, when Q is $-OR_5$, $R_5$ is $C_1$—$C_8$-alkyl; as well as the salts thereof.

10. Process for producing compounds of the formula I according to claim 1, which process comprises reacting, in the presence of an catalyst, a hydroquinone of the formula V

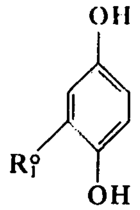

(V)

wherein $R_1^o$ is hydrogen or a group $R_1$ as defined in claim 1, with a functional alkylating agent capable of introducing a group of the formula II, the formula II having the meanings defined in claim 1.

11. Process according to claim 10, characterised in that a compound of the formula I in which q is 0 or 1 and p is 1 is produced by reaction of a compound of the formula V wherein $R_1^o$ is hydrogen, $C_1$—$C_8$-alkyl or a radical of the formula II with 0.1 to 1.0 mol of the alkylating agent per mol of the compound of the formula V.

12. Process according to claim 10, characterised in that compounds of the formula I in which q is 0 and p is 2 and both groups R of the formula II to be introduced are identical are produced by reaktion of a compound of the formula V with at least 2 mols of an alkylating agent per mol of the compound of the formula V, $R_1^o$ in the formula V being hydrogen.

13. Process for producing a hydroquinone of the formula I in which q is 1 and $R_1$ is $C_4$—$C_8$-alkyl, characterised in that a compound of the formula I wherein q is 0 and p is 1 is reacted with at least one mol of a $C_4$—$C_8$-olefin or -alcohol, in the presence of an acid catalyst.

14. Process according to claim 10, characterised in that the alkylating agent contains a reactive centre which is eliminated, transformed or rearranged during the alkylation reaction.

**Revendications**

1. Hydroquinones répondant à la formule I

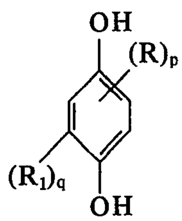

(I)

dans laquelle

p est égal à 1 ou à 2 et q est égal à 0 ou à 1, avec la condition que la somme (p + q) soit égale à 1 ou à 2,

R représente un radical de formule II

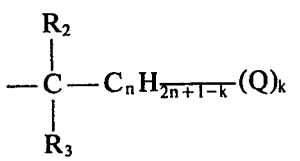

(II)

Q représente un radical $-COOR_4$, $-CONR_4R_5$, $-OR_5$, $-OCOR_7$, $-NR_8R_9$, $-PO(OR_{10})(O_xR_{11})$, $-SO_2OH$, $-SO_2Cl$, $-SO_2NR_5R_7$ ou $-CN$,

n représente un nombre entier de 1 à 20,

k représente le nombre 1 ou le nombre 2,

$R_1$ représente un alkyle en $C_1$—$C_8$ ou un radical de formule II, les symboles R et $R_1$ pouvant alors représenter des radicaux de formule II identiques ou différents,

$R_2$ et $R_3$ représentent chacun, indépendamment l'un de l'autre, un alkyle en $C_1$—$C_5$, et, lorsque Q est un radical $-COOR_4$, $R_2$ ou $R_3$ peut porter un radical $-COOR_4$, ou $R_2$ ou $R_3$ est fixé au radical $-C_nH_{2n+1-k}$ de telle façon qu'il se forme un radical cycloalkylène en $C_5$—$C_{12}$ porteur de k radicaux $-COOR_4$, les $R_4$ pouvant alors avoir des significations indentiques ou différentes.

$R_4$ représente l'hydrogène, un alkyle en $C_1$—$C_{20}$ qui peut être interrompu, de un à cinq fois, par un atome d'oxygène et/ou qui peut porter un radical $-OR_6$ dans lequel $R_6$ représente un alkyle en $C_1$—$C_{12}$, un cycloalkyle en $C_3$—$C_{12}$, un alcényle en $C_3$—$C_{20}$, un aralkyle en $C_7$—$C_{13}$ ou un aryle en $C_6$—$C_{10}$, l'aryle pouvant porter un ou deux radicaux alkyles en $C_1$—$C_4$, $R_4$ peut également représenter un alcényle en $C_3$—$C_{20}$, un cycloalkyle en $C_3$—$C_{12}$, un aralkyle en $C_7$—$C_{13}$, un aryle en $C_6$—$C_{10}$

éventuellement porteur d'un alkyle en $C_1$—$C_4$, un hétérocycle oxygéné à 5 ou 6 maillons, éventuellement porteur d'un ou deux alkyles en $C_1$—$C_4$, ou un méthyle porteur d'un noyau hétérocyclique oxygéné à 5 ou 6 maillons,

$R_5$ représente l'hydrogène ou un alkyle en $C_1$—$C_{20}$, ou

$R_4$ et $R_5$ forment ensemble, et avec l'atome d'azote auquel ils sont liés, un hétérocycle pentagonal ou hexagonal qui peut porter un ou deux alkyles en $C_1$—$C_4$,

$R_7$ représente l'hydrogène, un alkyle en $C_1$—$C_{20}$, un alcényleen $C_3$—$C_{20}$, un cycloalkyle en $C_3$—$C_{12}$, un aralkyle en $C_7$—$C_{13}$ ou un aryle en $C_6$—$C_{10}$ éventuellement porteur d'un ou deux alkyles en $C_1$—$C_4$,

$R_8$ représente l'hydrogène ou un alkyle en $C_1$—$C_{12}$, et

$R_9$ représente l'hydrogène, un alkyle en $C_1$—$C_{12}$ ou un radical $-COR_7$, ou encore

$R_8$ et $R_9$ forment ensemble, et avec l'atome d'azote auquel ils sont liés, un hétérocycle pentagonal ou hexagonal qui peut porter un ou deux alkyles en $C_1$—$C_4$,

x est égal à 0 ou à 1,

$R_{10}$ et $R_{11}$, lorsque x est égal à 1, représentent chacun, indépendamment l'un de l'autre, l'hydrogène ou un alkyle en $C_1$—$C_{20}$, ou $R_{10}$ et $R_{11}$ forment ensemble un alkylène en $C_2$ ou $C_3$ qui peut porter un ou plusieurs alkyles en $C_1$—$C_{20}$, et, lorsque x est égal à 0,

$R_{10}$ représente l'hydrogène ou un alkyle en $C_1$—$C_{20}$ et

$R_{11}$ représente un alkyle en $C_1$—$C_5$,

avec la condition que $R_1$ représente un radical de formule II lorsque Q désigne un radical $-SO_2OH$.

2. Composés de formule I suivant la revendication 1 dans lesquels p, q, R, $R_1$, $R_2$, $R_3$, $R_4$, $R_5$, $R_7$, Q, n et k ont les significations données à la revendication 1, $R_6$ représente un cycloalkyle en $C_3$—$C_{12}$, un alcényleen $C_3$—$C_{20}$, un aralkyle en $C_7$—$C_{13}$ ou un aryle en $C_6$—$C_{10}$, le radical aryle pouvant porter un ou deux alkyles en $C_1$—$C_4$, $R_8$ représente l'hydrogène ou un alkyle en $C_1$—$C_4$ et $R_9$ l'hydrogène, un alkyle en $C_1$—$C_4$ ou un radical $-COR_7$, ou $R_8$ et $R_9$ forment ensemble et avec l'atome d'azote auquel ils sont liés un hétérocycle à 5 ou 6 maillons qui peut porter un ou deux alkyles en $C_1$—$C_4$, et $R_{10}$ et $R_{11}$, lorsque x est égal à 1, représentent chacun, indépendamment l'un de l'autre, l'hydrogène ou un alkyle en $C_1$—$C_{20}$, ou $R_{10}$ et $R_{11}$ forment ensemble un alkylène en $C_2$ ou $C_3$ qui peut porter un ou plusiers alkyles en $C_1$—$C_{20}$, et $R_{10}$, lorsque x est égal à 0, représente un alkyle linéaire contenant de 1 à 5 atomes de carbone.

3. Composés de formule I selon la revendication 1 dans lesquels les radicaux R et $R_1$ occupent respectivement la position 2 et la position 5 du noyau de l'hydroquinone.

4. Composés selon la revendication 1 qui répondent à la formule III

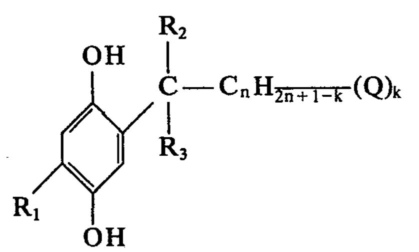

(III)

dans laquelle $R_2$, $R_3$, n, k et Q ont les significations données à la revendication 1, et $R_1$ représente un radical de formule II conforme à la définition donnée à la revendication 1 ou un radical de formule IV

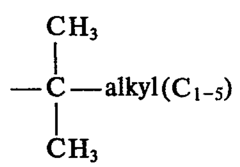

(IV)

ainsi qui les sels de ces composés.

5. Composés de formule III selon la revendication 4 dans lesquels $R_1$ a la signification donnée à la revendication 4, n et k ont les significations données à la revendication 1, Q représente un radical $-COOR_4$, $-CONR_4R_5$ ou $-OR_5$, les symboles $R_4$ et $R_5$ ayant les significations données à la revendication 1, $R_2$ et $R_3$ représentent chacun, indépendamment l'un de l'autre, un radical méthyle, éthyle, n-propyle, isopropyle néopentyle, ou $R_2$ ou $R_3$ peut porter un radical $-COOR_4$, ou $R_2$ ou $R_3$ est fixé au radical $-C_nH_{2n+1-k}$ de telle façon qu'il se forme un radical cycloalkylène en $C_5$—$C_8$ porteur d'un radical $-COOR_4$, ainsi que les sels de ces composés.

6. Composés de formule III selon la revendication 5 dans lesquels k représente le nombre 1, $R_1$ a la signification donnée à la revendication 4, Q représente un radical $-COOR_4$, $-CONR_4R_5$ ou $-OR_5$, n désigne un nombre de 1 à 10 $R_2$ et $R_3$ représentent chacun, indépendamment l'un de l'autre, un radical méthyle, éthyle ou néopentyle, ou $R_2$ ou $R_3$ est fixé au radical $-C_nH_{2n+1-k}$ de telle façon qu'il se forme un radical cyclohexyle porteur d'un radical $-COOR_4$, $R_4$ représente l'hydrogène ou un alkyle en $C_1$—$C_{20}$ qui

peut être interrompu par un à trois atomes d'oxygène et/ou porter un radical $-OR_6$, le symbole $R_6$ désignant un radical cyclopentyle, cyclohexyle, cyclo-octyle, alcényle en $C_3-C_{10}$, phényle, benzyle, phényléthyle, benzhydryle ou naphtylméthyle, $R_4$ représente en outre un alcényle en $C_3-C_{15}$, un phényle éventuellement porteur d'un ou de deux alkyles en $C_1-C_4$, un benzyle, un phényléthyle, un cyclopentyle, un cyclohexyle ou un radical hétérocyclique oxygéné à 5 ou 6 maillons qui peut porter un ou deux alkyles en $C_1-C_4$, et $R_5$ a la signification donnée à la revendication 1, ou $R_4$ et $R_5$ forment ensemble et avec l'atome d'azote auquel ils sont liés un noyau hétérocyclique pentagonal ou hexagonal qui peut porter un ou deux alkyles en $C_1-C_4$, ainsi que les sels de ces composés.

7. Composés de formule III selon la revendication 6 dans lesquels $R_1$, k, n, $R_2$ et $R_3$ ont les significations données à la revendication 6, Q représente un radical $-COOR_4$, $-CONR_4R_5$ ou $-OR_5$, $R_4$ représente l'hydrogène, un alkyle en $C_1-C_{20}$ qui peut être interrompu par 1 ou 2 atomes d'oxygène et/ou porter un radical cyclohexyloxy, alcényloxy en $C_3-C_{20}$, phénoxy ou benzyloxy, $R_4$ représente en outre un alcényle en $C_3-C_{15}$, un phényle, un benzyle, un phényléthyle, un cyclopentyle, un cyclohexyle ou un noyau hétérocyclique oxygéné à 5 ou 6 maillons, et $R_5$ représente l'hydrogène ou un alkyle en $C_1-C_{15}$, ou encore $R_4$ et $R_5$ forment ensemble et avec l'atome d'azote auquel ils sont liés un noyau hétérocyclique à 5 ou 6 maillons qui peut porter un ou deux alkyles en $C_1-C_4$.

8. Composés de formule III selon la revendication 7 dans lesquels k, $R_1$, Q, n, $R_2$, $R_3$ et $R_5$ ont les significations données à la revendication 7, et $R_4$ représente l'hydrogène ou un alkyle en $C_1-C_{16}$ qui peut être interrompu par un atome d'oxygène et/ou porter un phénoxy, $R_4$ représente en outre un alcényle en $C_3-C_{15}$, un phényle, un benzyle, un tétrahydrofurannyle-3, un tétrahydropyrannyle-4 ou un tétrahydrofurfuryle, ou encore $R_4$ et $R_5$ forment ensemble et avec l'atome d'azote auquel ils sont liés un noyau hétérocyclique à 5 ou 6 maillons qui peut porter un ou deux alkyles en $C_1-C_4$, ainsi que les sels de ces composés.

9. Composés de formule III selon la revendication 8 dans lesquels k représente le nombre 1, $R_1$ a la signification donnée à la revendication 4, Q représente un radical $-COOR_4$, $-CONR_4R_5$ ou $-OR_5$, n représente un nombre de 1 à 3, $R_2$ et $R_3$ représente chacun un méthyle ou un néopentyle, $R_4$ représente l'hydrogène, un alkyle en $C_1-C_{18}$ qui peut être interrompu par un atome d'oxygène ou porter un phénoxy, $R_4$ représente en outre un radical phényle, benzyle ou tétrahydrofurannyle-3, $R_5$ représente l'hydrogène ou un alkyle en $C_1-C_{15}$, ou $R_4$ et $R_5$ forment ensemble et avec l'atome d'azote auquel ils sont liés un noyau hétérocyclique à 5 ou 6 maillons qui peut porter un ou deux alkyles en $C_1-C_4$, avec la condition que, lorsque Q représente un radical $-OR_5$, le symbole $R_5$ représente un alkyle en $C_1-C_8$, ainsi que les sels de ces composés.

10. Procédé de préparation de composés de formule I selon la revendication 1, procédé caractérisé en ce qu'on fait réagir, en présence d'un catalyseur acide, une hydroquinone répondant à la formule V

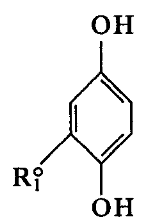

(V)

dans laquelle $R_1^o$ représente l'hydrogène ou a la signification donnée par $R_1$ à la revendication 1, avec un agent d'alkylation fonctionnel capable d'introduire un radical de formule II tel que défini à la revendication 1.

11. Procédé selon la revendication 10 caractérisé en ce qu'on prépare un composé de formule I dans lequel q représente le nombre 0 ou le nombre 1 et p le nombre 1, par réaction d'un composé de formule V avec de 0,1 à 1,0 mol d l'agent d'alkylation par mole du composé de formule V, le symbole $R_1^o$ présent dans la formule V désignant l'hydrogène, un alkyle en $C_1-C_8$ ou un radical de formule II.

12. Procédé selon la revendication 10 caractérisé en ce qu'on prépare des composés de formule I dans lesquels q représente le nombre 0 et p le nombre 2, et dans lesquels les deux radicaux R de formule II a introduire sont identiques, par réaction d'un composé de formule V avec au moins 2 mol d'un agent d'alkylation par mole du composé de formule V dans lequel $R_1^o$ représente l'hydrogène.

13. Procédé de préparation d'une hydroquinone de formule I dans laquelle q représente le nombre 1 et $R_1$ représente un alkyle en $C_4-C_8$, procédé caractérisé en ce qu'on fait réagir un composé de formule I dans lequel q représente le nombre 0 et p le nombre 1 avec au moins 1 mol d'un alcool ou d'une oléfine en $C_4-C_8$, en présence d'un catalyseur acide.

14. Procédé selon la revendication 10 caractérisé en ce que l'agent d'alkylation contient un centre actif qui est éliminé, modifié ou transposé au cours de la réaction d'alkylation.